(19) Europäisches Patentamt
European Patent Office
Office européen des brevets

(11) **EP 0 643 045 B1**

(12) **EUROPÄISCHE PATENTSCHRIFT**

(45) Veröffentlichungstag und Bekanntmachung des Hinweises auf die Patenterteilung:
**08.03.2000 Patentblatt 2000/10**

(51) Int Cl.[7]: **C07D 215/14**, A61K 31/47, C07D 215/18, C07D 215/20, C07D 221/16

(21) Anmeldenummer: **94810504.4**

(22) Anmeldetag: **01.09.1994**

(54) **Chinolin-Verbindungen als Leukotriene Antagonisten**

Quinoline derivatives as leukotriene antagonists

Dérivés de quinoléine comme antagonistes de leukotriènes

(84) Benannte Vertragsstaaten:
**AT BE CH DE DK ES FR GB GR IE IT LI LU NL PT SE**

(30) Priorität: **10.09.1993 EP 93810645**

(43) Veröffentlichungstag der Anmeldung:
**15.03.1995 Patentblatt 1995/11**

(73) Patentinhaber:
- **Novartis AG**
  **4058 Basel (CH)**
  Benannte Vertragsstaaten:
  **BE CH DE DK ES FR GB GR IE IT LI LU NL PT SE**
- **Novartis-Erfindungen Verwaltungsgesellschaft m.b.H.**
  **1235 Wien (AT)**
  Benannte Vertragsstaaten:
  **AT**

(72) Erfinder:
- **von Sprecher, Andreas, Dr.**
  **CH-4104 Oberwil (CH)**
- **Beck, Andreas, Dr.**
  **D-79108 Freiburg (DE)**
- **Gerspacher, Marc, Dr.**
  **CH-5200 Brugg (CH)**

(56) Entgegenhaltungen:
**EP-A- 0 355 353**

- **JOURNAL OF MEDICINAL CHEMISTRY, Bd.30, Nr.2, 1987, WASHINGTON US Seiten 400 - 405 JH. H. MUSSER ET AL. 'Leukotriene D4 antagonists and 5-lipoxygenase inhibitors. Synthesis of...'**
- **JOURNAL OF MEDICINAL CHEMISTRY, Bd.35, Nr.7, 1992, WASHINGTON US Seiten 1200 - 1209 J. SCOTT SAWYER ET AL. 'Optimization of the quinoline and substituted benzyl moieties of a series of phenyltetrazole leukotriene D4 receptor antagonists'**
- **JOURNAL OF MEDICINAL CHEMISTRY, Bd.35, Nr.23, 1992, WASHINGTON US Seiten 4306 - 4314 R. LABAUDINI RE ET AL. 'Omega((4-phenyl-2-quinolyl)oxy)alkanoic acid derivatives: ...'**
- **JOURNAL OF MEDICINAL CHEMISTRY, Bd.35, Nr.14, 1992, WASHINGTON US Seiten 2501 - 2524 JH. MUSSER ET AL. '5-Lipoxygenase: properties, pharmacology, and the quinolinyl(bridged)aryl class of inhibitors'**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 643 045 B1

## Beschreibung

Die Erfindung betrifft Verbindungen der Formel I,

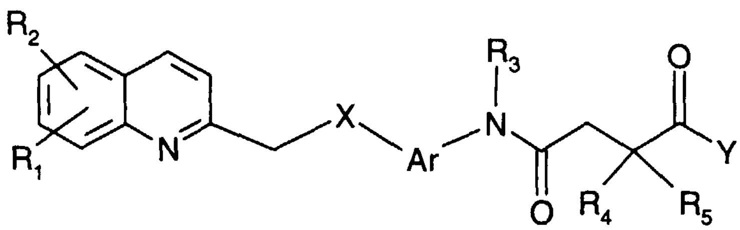

(I)

worin

$R_1$ und $R_2$ unabhängig voneinander Wasserstoff, Niederalkyl, Halogen-niederalkyl, Halogen, Niederalkoxy, oder Halogen-niederalkoxy bedeuten, oder

$R_1$ und $R_2$ zusammen $-(CH_2)_m-$, worin m für 3, 4 oder 5 steht, bedeuten,

$R_3$ Wasserstoff, Niederalkyl, oder (Carboxy- oder Niederalkoxy carbonyl-)phenyl-niederalkyl, welches im Phenylring noch zusätzlich durch Niederalkoxy substituiert sein kann, bedeutet,

$R_4$ und $R_5$ unabhängig voneinander Niederalkyl bedeuten, oder

$R_4$ und $R_5$ zusammen $-(CH_2)_n-$, worin n für 3, 4, 5 oder 6 steht, bedeuten,

X für O oder S steht,

Ar für 1,3-Phenylen oder 2,7-Naphthylen steht, und

Y Hydroxy oder Niederalkoxy bedeutet;

und Salze davon, Verfahren zur Herstellung dieser Verbindungen, pharmazeutische Präparate, die diese Verbindungen enthalten, die Verwendung dieser Verbindungen zur therapeutischen Behandlung des menschlichen oder tierischen Körpers oder zur Herstellung pharmazeutischer Präparate.

Die vor- und nachstehend verwendeten Allgemeinbegriffe haben im Rahmen der vorliegenden Anmeldung vorzugsweise die folgenden Bedeutungen:

Das Präfix "Nieder" bezeichnet einen Rest bis und mit 7 und insbesondere bis und mit 4 Kohlenstoffatomen.

Niederalkyl ist z.B. n-Propyl, Isopropyl, n-Butyl, Isobutyl, sek-Butyl, tert-Butyl, n-Pentyl, Neopentyl, n-Hexyl oder n-Heptyl, vorzugsweise Ethyl und vor allem Methyl.

$C_1$-$C_3$-Alkyl steht für Methyl, Ethyl, n-Propyl und Isopropyl.

Niederalkyl als Bedeutung von R4 und $R_5$ ist bevorzugt Ethyl.

Halogen-niederalkyl ist z.B. Trifluormethyl.

Halogen-niederalkoxy ist z.B. Trifluormethoxy.

Cycloalkyl ist vorzugsweise $C_3$-$C_8$- und insbesondere $C_5$-$C_6$-Cycloalkyl, was bedeuten soll, dass es 3 bis 8 bzw. 5 bis 6 Ringkohlenstoffe enthält. Cycloalkyl kann aber auch substituiert sein, z.B. durch Niederalkyl.

Halogen steht insbesondere für Chlor oder Brom, vor allem für Fluor, kann aber auch Iod bedeuten.

Acyl steht z.B. für Niederalkanoyl; Niederalkanoyl ist z.B. Acetyl, Propionyl oder Pivaloyl, aber etwa auch Formyl.

Aminocarbonyl ist die Gruppe $-CONH_2$.

Stehen $R_1$ und $R_2$ zusammen für den bivalenten Rest $-(CH_2)_m-$, so ist dieser bivalente Rest vorzugsweise an zwei benachbarten C-Atomen des Phenylrings angeknüpft (z.B. als 6,7-Cyclopentanochinolinyl).

Stehen $R_4$ und $R_5$ zusammen für den bivalenten Rest $-(CH_2)_n-$, so ist dieser bivalente Rest zweimal am selben C-Atom angeknüpft, so dass ein Cycloalkan gebildet wird.

Aryl ist z.B. Phenyl, das unsubstituiert oder substituiert ist. Aryl ist bevorzugt Phenyl, das unsubstituiert oder

durch einen oder mehrere, insbesondere einen oder zwei, Substituenten aus der Gruppe bestehend aus Niederalkyl, Niederalkoxy, Niederalkenyloxy, Hydroxy, Niederalkanoyloxy, Nitro, Amino, Halogen, Trifluormethyl, Carboxy, Niederalkoxycarbonyl, Aminocarbonyl, N-Niederalkylaminocarbonyl, N,N-Diniederalkylaminocarbonyl, Cyano, Niederalkanoyl, Benzoyl und Niederalkylsulfonyl substituiert ist. In erster Linie bedeutet Aryl Phenyl, das unsubstituiert oder durch Niederalkyl, Niederalkoxy, Niederalkenyloxy, Hydroxy, Halogen oder Trifluormethyl substituiert ist, und vor allen Dingen Phenyl.

1,3-Phenylen und 2,7-Naphthylen stehen für die bivalenten Reste

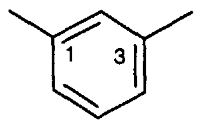 bzw. 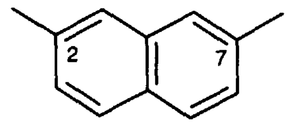

Salze von Verbindungen der Formel I sind insbesondere pharmazeutisch verwendbare Salze, vor allem Salze mit Basen, wie entsprechende Alkalimetall- oder Erdalkalimetallsalze, z.B. Natrium-, Kalium- oder Magnesiumsalze, pharmazeutisch verwendbare Uebergangsmetallsalze, wie Zink- oder Kupfersalze, oder Salze mit Ammoniak oder organischen Aminen, wie cyclischen Aminen, wie Mono-, Di- oder Triniederalkylaminen, wie Hydroxyniederalkylaminen, z. B. Mono-, Di- oder Trihydroxyniederalkylaminen, Hydroxyniederalkyl-niederalkyl-aminen oder Polyhydroxyniederalkylaminen. Cyclische Amine sind z.B. Morpholin, Thiomorpholin, Piperidin oder Pyrrolidin. Als Mononiederalkylamine kommen beispielsweise Ethyl- und t-Butylamin, als Diniederalkylamine beispielsweise Diethyl- und Diisopropylamin und als Triniederalkylamine beispielsweise Trimethyl- und Triethylamin in Betracht. Entsprechende Hydroxyniederalkylamine sind z.B. Mono-, Di- und Triethanolamin; Hydroxyniederalkyl-niederalkyl-amine sind z.B. N,N-Dimethylamino- und N,N-Diethylamino-ethanol; als Polyhydroxyniederalkylamin kommt z.B. Glucosamin in Frage. In bestimmten Fällen können auch Säureadditionssalze, beispielsweise mit starken anorganischen Säuren, wie Mineralsäuren, z.B. Schwefelsäure, einer Phosphorsäure oder einer Halogenwasserstoffsäure, mit starken organischen Carbonsäuren, wie Niederalkancarbonsäuren, z.B. Essigsäure, wie gegebenenfalls ungesättigten Dicarbonsäuren, z.B. Malon-, Malein- oder Fumarsäure, oder wie Hydroxycarbonsäuren, z.B. Wein- oder Zitronensäure, oder mit Sulfonsäuren, wie Niederalkan- oder gegebenenfalls substituierten Benzolsulfonsäuren, z.B. Methan- oder p-Toluolsulfonsäure, gebildet werden. Verbindungen der Formel I mit einer sauren Gruppe, z.B. Carboxy, und einer basischen Gruppe, z.B. Amino, können z. B. auch in Form von inneren Salzen, d.h. in zwitterionischer Form vorliegen, oder es kann ein Teil des Moleküls als inneres Salz, und ein anderer Teil als normales Salz vorliegen. Umfasst sind ferner für pharmazeutische Verwendungen ungeeignete Salze, da diese beispielsweise für die Isolierung bzw. Reinigung von freien Verbindungen I sowie von deren pharmazeutisch verwendbaren Salzen verwendet werden können.

Die Verbindungen der Formel I weisen wertvolle pharmakologische Eigenschaften, insbesondere eine ausgeprägte antagonistische Wirkung gegenüber Leukotrienen, auf.

So hemmen sie z.B. in vitro mit einem $IC_{50}$-Wert von etwa 0,0001 bis etwa 0,05 mmol/l die durch Leukotrien $D_4$ ($LTD_4$) induzierte Kontraktion des Meerschweinchen-Dünndarms. Diese als $LTD_4$-Antagonismus bezeichnete Wirkung kann experimentell z.B. verifiziert werden, indem an Meerschweinchen-Ileum-Segmenten in einem Organbad [Standard methode: Tyrode-Lösung bei 38°C unter Begasung mit Oxicarbon (Mischung aus Sauerstoff und $CO_2$) unter Belastung von 1 g] mit synthetischem Leukotrien $D_4$ (in Form des Kaliumsalzes) Kontraktionen ausgelöst und diese isotonisch registriert werden. Das Ausmass der Hemmung der Kontraktionen durch die Prüfsubstanz wird nach einer Vorinkubation von 2 Minuten in Form der $IC_{50}$-Werte ermittelt, wobei als $IC_{50}$ diejenige Konzentration bezeichnet wird, bei welcher die Testkontraktionen um 50 % reduziert sind.

Die Verbindungen der Formel I sind auch in vivo ausgezeichnet wirksam. So kann z.B. im Bronchospasmus-Standardtest am Meerschweinchen nach Verabreichung der Testsubstanz als Aerosol ein deutlicher antagonistischer Effekt gegenüber $LTD_4$ beobachtet werden ($ED_{50}$ von etwa 0,0003 bis etwa 0.05 % w/v Spray/min). In diesem Testmodell werden narkotisierte Meerschweinchen (Urethan 1,4 g/kg) in einem Plethysmograph installiert. Oesophagaler Druck und Atemfluss werden mit entsprechender Computerunterstützung in verschiedene Lungenparameter, z.B. Compliance, umgewandelt. Nach einer kurzen Stabilisationsphase erfolgt die Applikation der Prüfsubstanz mit einem Monaghan-Ultraschall-Sprühgerät. Das produzierte Aerosol (Vehikel oder Wirkstoff) wird von den spontanatmenden Tieren während 1 Minute via Tracheakanüle inhaliert. Nach Ablauf einer bestimmten Behandlungszeit wird mit einem zweiten identischen Inhalationssystem während 2 Minuten $LTD_4$ verabreicht. Die Abnahme der Compliance dient als Ausmass der Stärke der von $LTD_4$ ausgelösten Bronchokonstriktion. Die Mittelwerte der Behandlungsgruppe werden mit den Mittelwerten der Kontrolltiere verglichen. Die Wirksamkeit der Prüfsubstanz wird nach folgender Formel errechnet:

$$\% \text{ Hemmung} = 100 - \frac{(100 - \text{Compliance Präparat}) \bullet 100}{(100 - \text{Compliance Kontrolle})}$$

und die $IC_{50}$-Werte durch lineare Regressionsanalyse ermittelt.

Ganz besonders zeichnen sich die Verbindungen der Formel I bei oraler Verabreichung aus, und zwar durch ihre hohe Wirkungsaktivität und lange Wirkungsdauer. Zum Beispiel werden die Testsubstanzen den Tieren - mit der gleichen Versuchsanordnung wie oben beim Bronchospasmus-Standardtest beschrieben - als Suspension in Methylcellulose mittels Magensonde verabreicht. Bei Behandlungszeiten bis zu 8 Stunden wird im Bereich von etwa 0,003 bis 1,0 mg/kg eine markante Reduktion der durch $LTD_4$ erzeugten Bronchokonstriktion beobachtet.

Exzellente Wirkungen zeigen die Verbindungen der Formel I auch im durch Leukotrien $E_4$ ($LTE_4$) induzierten Bronchospasmus, zum Beispiel bei oraler Verabreichung. Bei Behandlungszeiten bis zu 8 Stunden wird im Bereich von etwa 0,001 bis 1,0 mg/kg p.o. wiederum eine starke Wirksamkeit beobachtet.

Die Verbindungen der Formel I können daher z.B. überall dort therapeutische Anwendung finden, wo die Wirkung der Leukotriene zu krankhaften Zuständen führt, und diese mildern oder beheben. Die Leukotriene spielen eine wichtige Rolle u.a. bei der Entstehung von allergischen und entzündlichen Prozessen. Demzufolge können die Verbindungen der Formel I z.B. als Antiallergika verwendet werden, etwa zur Behandlung von allergischen Zuständen und Erkrankungen, wie insbesondere Asthma, aber auch z.B. bei Heufieber oder obstruktiven Lungenkrankheiten. Die Verbindungen der Formel I können z.B. auch zur Behandlung von entzündlichen Erkrankungen der Lunge und anderer Organe verwendet werden, z.B. bei zystischer Fibrose oder Schocklunge (Adult Respiratory Distress Syndrome), aber auch z.B. bei Psoriasis, Colitis ulcerosa oder Morbus Crohn, bei septischem Schock oder entzündlichen Erkrankungen des Auges.

John H. Musser et al., J. Med. Chem. 1987, 30, 400-405, beschreiben verschiedene Verbindungen mit Wirkung als Leukotrien-Antagonisten. Diese Verbindungen umfassen 4-[3-(2-Chinolinylmethoxy)-phenylhydroxyamino]-4-oxobutansäuremethylester und 4-[3-(2-Chinolinylmethoxy)-phenylamino]-4-oxobutansäuremethylester. Musser et al. stellen fest, es habe sich bereits früher gezeigt, dass die erste der beiden Verbindungen mutagen sei; experimentelle Untersuchungen zeigten, dass auch die zweite Verbindung mutagen ist.

EP-A-0355353 beschreibt Cycloalkythiazol-Derivate von 4-Phenylamino-4-oxobutansäuren und gibt vergleichende $LTD_4$-Bindungsdaten für Cyclopropylthiazolyl-Derivate einer 2,2-Diethyl-substituierten 4-Phenylamino-4-oxobutansäure und der entsprechenden 2,2-unsubstituierten Säure an.

Die Erfindung betrifft insbesondere die Verbindungen der Formel I, worin $R_1$ und $R_2$ unabhängig voneinander Wasserstoff, Niederalkyl, Halogen-niederalkyl, Halogen oder Niederalkoxy bedeuten, $R_3$ Wasserstoff, Niederalkyl, oder (Carboxy- oder Niederalkoxycarbonyl-)phenyl-niederalkyl bedeutet, $R_4$ und $R_5$ unabhängig voneinander Niederalkyl bedeuten, oder $R_4$ und $R_5$ zusammen $-(CH_2)_4-$ oder $-(CH_2)_5-$ bedeuten, X für O oder S steht, Ar für 1,3-Phenylen oder 2,7-Naphthylen steht, und Y Hydroxy oder Niederalkoxy bedeutet; und Salze davon.

Die Erfindung betrifft vorzugsweise die Verbindungen der Formel I, worin $R_1$ und $R_2$ unabhängig voneinander Wasserstoff, Niederalkyl, Halogen-niederalkyl, Halogen, Niederalkoxy, oder Halogen-niederalkoxy bedeuten, oder $R_1$ und $R_2$ zusammen $-(CH_2)_3-$ oder - $(CH_2)_4-$ bedeuten, $R_3$ Wasserstoff, Niederalkyl, oder (Carboxy oder Niederalkoxycarbonyl) phenyl-niederalkyl, welches im Phenylring noch zusätzlich durch Niederalkoxy substituiert sein kann, bedeutet, $R_4$ und $R_5$ unabhängig voneinander Niederalkyl bedeuten, oder $R_4$ und $R_5$ zusammen $-(CH_2)_4-$ oder $-(CH_2)_5-$ bedeuten, X für O oder S steht, Ar für 1,3-Phenylen oder 2,7-Naphthylen steht, und Y Hydroxy oder Niederalkoxy bedeutet; und Salze davon.

Die Erfindung betrifft bevorzugt die Verbindungen der Formel I, worin $R_1$ und $R_2$ unabhängig voneinander Wasserstoff, Niederalkyl, Trifluormethyl, Halogen, oder Niederalkoxy bedeuten, $R_3$ Wasserstoff, Niederalkyl, oder (Carboxy oder Niederalkoxycarbonyl)phenyl-niederalkyl bedeutet, $R_4$ und $R_5$ unabhängig voneinander Niederalkyl bedeuten, oder $R_4$ und $R_5$ zusammen $-(CH_2)_4-$ oder $-(CH_2)_5-$ bedeuten, X für O oder S steht, Ar für 1,3-Phenylen oder 2,7-Naphthylen steht, und Y Hydroxy oder Niederalkoxy bedeutet; und Salze davon.

Die Erfindung betrifft in erster Linie die Verbindungen der Formel I, worin $R_1$ und $R_2$ unabhängig voneinander Wasserstoff, Niederalkyl, Trifluormethyl, Halogen, oder Niederalkoxy bedeuten, $R_3$ für Wasserstoff, Niederalkyl, oder Carboxyphenyl-niederalkyl steht, $R_4$ und $R_5$ unabhängig voneinander Niederalkyl bedeuten, oder $R_4$ und $R_5$ zusammen-$(CH_2)_4-$ oder $-(CH_2)_5-$ bedeuten, X für O oder S steht, Ar für 1,3-Phenylen oder 2,7-Naphthylen steht, und Y Hydroxy oder Niederalkoxy bedeutet, und Salze davon.

Die Erfindung betrifft vor allem die Verbindungen der Formel I, worin $R_1$ Wasserstoff, Niederalkyl, Trifluormethyl, Halogen, oder Niederalkoxy bedeutet, $R_2$ und $R_3$ für Wasserstoff stehen, $R_4$ und $R_5$ unabhängig voneinander $C_1$-$C_3$-Alkyl bedeuten, oder $R_4$ und $R_5$ zusammen--$(CH_2)_4-$ oder $-(CH_2)_5-$ bedeuten, X für O oder S steht, Ar für 1,3-Phenylen oder 2,7-Naphthylen steht, und Y Hydroxy bedeutet, und pharmazeutisch verwendbare Salze davon.

Eine besonders bevorzugte Gruppe innerhalb der Verbindungen der Formel I sind die Verbindungen der Formel Ia,

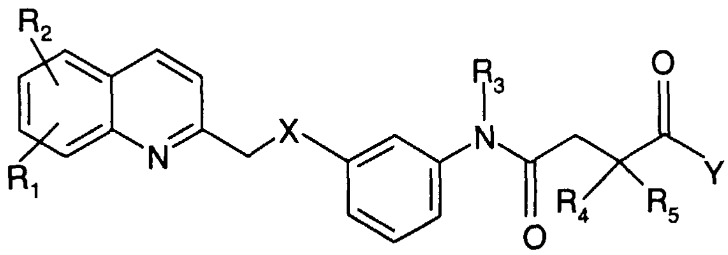

(Ia)

worin $R_1$ und $R_2$ unabhängig voneinander Wasserstoff, Chlor oder Fluor bedeuten, $R_3$ für Wasserstoff steht, $R_4$ und $R_5$ Ethyl bedeuten, X für O steht und Y Hydroxy oder Niederalkoxy bedeutet, und pharmazeutisch verwendbare Salze davon.

In erster Linie bevorzugt sind die Verbindungen der Formel Ia, worin $R_1$ Wasserstoff, Chlor oder Fluor bedeutet, $R_2$ für Wasserstoff oder Fluor steht, $R_3$ Wasserstoff bedeutet, $R_4$ und $R_5$ Ethyl bedeuten, X für O steht und Y Hydroxy bedeutet, und pharmazeutisch verwendbare Salze davon.

Die Erfindung betrifft vor allem die in den Beispielen beschriebenen spezifischen Verbindungen und Salze davon.

Die Verbindungen der Formel I können in an sich bekannter Weise hergestellt werden, indem man z.B.

(a) eine Verbindung der Formel II,

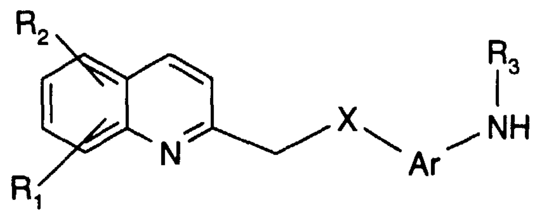

(II)

worin $R_1$,$R_2$,$R_3$, X und Ar wie unter Formel I definiert sind, mit einer Verbindung der Formel III,

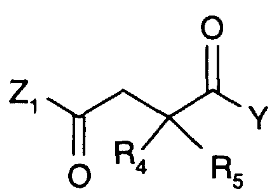

(III)

worin die Gruppe -$COZ_1$ Carboxy oder ein reaktionsfähiges Carboxyderivat bedeutet und $R_4$, $R_5$ und Y wie unter Formel I definiert sind, umsetzt, oder

(b) eine Verbindung der Formel IV,

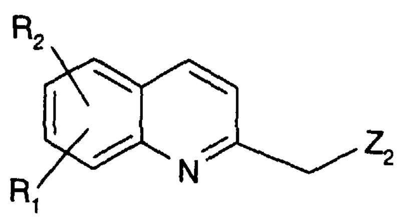

(IV)

worin $Z_2$ eine nukleofuge Abgangsgruppe bedeutet und $R_1$ und $R_2$ wie unter Formel I definiert sind, mit einer Verbindung der Formel V,

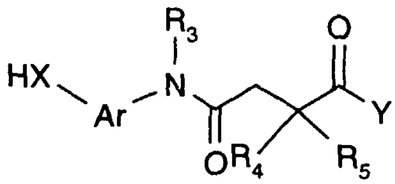

(V)

worin $R_3$,$R_4$,$R_5$, X, Ar und Y wie unter Formel I definiert sind, oder einem Salz davon, umsetzt,

und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt.

Verfahren (a): Die Umsetzung gemäss dem Verfahren (a) entspricht der an sich bekannten Acylierung von primären oder sekundären aromatischen Aminen.

Ein reaktionsfähiges Carboxyderivat $-COZ_1$ ist z.B. ein Carbonsäurehalogenid, etwa ein Säurechlorid, ein Carbonsäureimidazolid, ein Carbonsäureanhydrid oder ein reaktiver Carbonsäureester, z.B. ein p-Nitrophenylester.

Zur Herstellung von Verbindungen der Formel I mit Y = OH kann vorteilhaft als Verbindung der Formel III auch ein inneres Säureanhydrid eingesetzt werden; ein solches inneres Säureanhydrid entspricht einer Verbindung der Formel III, worin die Reste $Z_1$ und Y zusammen eine -O- Brücke bilden. Es kann aus der entsprechenden Dicarbonsäure durch Umsetzung z.B. mit Oxalylchlorid oder Acetylchlorid hergestellt werden.

Eine vorteilhafte Methode zur Herstellung von Verbindungen der Formel I mit Y = Niederalkoxy - insbesondere auch für solche mit einem voluminösen Rest $R_3$ ($R_3$ = Niederalkyl, substituiertes Niederalkyl) - besteht darin, eine Verbindung der Formel II mit einer Verbindung der Formel III, worin Y Niederalkoxy und $Z_1$ Carboxy ist, (Halbester) in Gegenwart z.B. von N-(3-Dimethylaminopropyl)-N'-ethylcarbodiimid-hydrochlorid und 4-Dimethylaminopyridin umzusetzen.

Die Verbindungen der Formel II, worin X für O oder S steht, werden z.B. dadurch hergestellt, dass man eine Verbindung der Formel IV [s. Verfahren (b)] mit einer Verbindung der Formel VI,

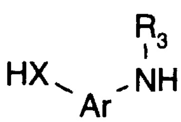

(VI)

worin X O oder S ist, umsetzt.

Die Verbindungen der Formel III sind an sich bekannt oder werden analog zu den bekannten Verbindungen hergestellt.

Verfahren (b): Das Verfahren (b) dient vorzugsweise der Herstellung von Verbindungen der Formel I, worin X für O oder S steht. Dabei wird eine Hydroxy- bzw. Mercaptoarylverbindung der Formel V in an sich bekannter Weise mit einem Chinolinmethyl- Derivat der Formel IV alkyliert.

In einer Verbindung der Formel IV bedeutet die nucleofuge Abgangsgruppe Z2 z.B. Halogen, insbesondere Brom, Iod oder Chlor, oder Sulfonyloxy, z.B. Methyl- oder p-Toluolsulfonyloxy.

Die Verbindungen der Formel IV werden in an sich bekannter Weise beispielsweise aus den entsprechenden Methylchinolinen hergestellt, z.B. durch Halogenierung, etwa mit N-Bromsuccinimid.

Die Verbindungen der Formel V werden z.B. analog zu den Verbindungen der Formel I in Verfahren (a) hergestellt, wobei man anstelle einer Verbindung der Formel II eine Verbindung der Formel VI [s. Verfahren (a)] als Ausgangsmaterial einsetzt.

Verbindungen der Formel I können auch in andere Verbindungen der Formel I überführt werden.

So kann z.B. eine Verbindung der Formel I, worin $R_3$ für Wasserstoff steht, am Amidstickstoff alkyliert werden, wobei man eine Verbindung der Formel I, worin $R_3$ Niederalkyl oder substituiertes Niederalkyl bedeutet, erhält. Die Umsetzung wird in Gegenwart einer geeigneten Base, z.B. NaH oder Natriummethoxid, durchgeführt, und als Alkylierungsmittel dient z.B. eine Verbindung $R_3$-$Z_3$, worin $Z_3$ eine nukleofuge Abgangsgruppe, z.B. Halogen, insbesondere Brom, Iod oder Chlor, oder Sulfonyloxy, z.B. Methyl- oder p-Toluolsulfonyloxy, bedeutet und $R_3$ wie unter Formel I definiert ist.

Carbonsäuren der Formel I, d.h. Verbindungen der Formel I mit Y = OH, können in an sich bekannter Weise in die entsprechenden Carbonsäure-Derivate der Formel I, worin Y für Niederalkoxy steht, umgewandelt werden. Umgekehrt können die genannten Carbonsäure-Derivate in an sich bekannter Weise, z.B. durch Umsetzung mit LiOH/ Tetrahydrofuran/Wasser/Methanol, /Methanol, etwa bei einer Temperatur von 50°, in die freien Carbonsäuren der Formel I überführt werden.

Wenn irgendwelche Zwischenprodukte störende reaktionsfähige Gruppen, z.B. Carboxy-, Hydroxy-, Mercapto- oder Aminogruppen, enthalten, können diese vorübergehend durch leichtabspaltbare Schutzgruppen geschützt werden. Die Auswahl von geeigneten Schutzgruppen, ihre Einführung und Entfernung sind an sich bekannt und sind z.B. in J.F.W. McOmie, Protective Groups in Organic Chemistry, Plenum Press, London, New York 1973 beschrieben.

Salze von Verbindungen I können in an sich bekannter Weise hergestellt werden. So erhält man beispielsweise Säureadditionssalze von Verbindungen I durch Behandeln mit einer geeigneten Säure oder einem geeigneten Ionenaustauscherreagens und Salze mit Basen durch Behandeln mit einer geeigneten Base oder einem geeigneten Ionenaustauscherreagens. Salze von Verbindungen der Formel I können in üblicher Weise in die freien Verbindungen I überführt werden, Säureadditionssalze z.B. durch Behandeln mit einem geeigneten basischen Mittel oder einem geeigneten Ionenaustauscherreagens und Salze mit Basen z.B. durch Behandeln mit einer geeigneten Säure oder einem geeigneten Ionenaustauscherreagens.

Salze von Verbindungen I können in an sich bekannter Weise in andere Salze von Verbindungen I umgewandelt werden, Säureadditionssalze beispielsweise in andere Säureadditionssalze, z.B. durch Behandeln eines Salzes einer anorganischen Säure, wie eines Hydrochlorids, mit einem geeigneten Metallsalz, wie einem Natrium-, Barium- oder Silbersalz, einer Säure, z.B. mit Silberacetat, in einem geeigneten Lösungsmittel, in welchem ein sich bildendes anorganisches Salz, z.B. Silberchlorid, unlöslich ist und damit aus dem Reaktionsgemisch ausscheidet.

Je nach Verfahrensweise bzw. Reaktionsbedingungen können die Verbindungen I mit salzbildenden Eigenschaften in freier Form oder in Form von Salzen erhalten werden.

Infolge der engen Beziehung zwischen der Verbindung I in freier Form und in Form ihrer Salze sind im vorausgegangenen und nachfolgend unter der freien Verbindung I bzw. ihren Salzen sinn- und zweckgemäss gegebenenfalls auch die entsprechenden Salze bzw. die freie Verbindung I zu verstehen.

Die Verbindungen I einschliesslich ihrer Salze von salzbildenden Verbindungen können auch in Form ihrer Hydrate erhalten werden und/oder andere, beispielsweise gegebenenfalls zur Kristallisation von in fester Form vorliegenden Verbindungen verwendete, Lösungsmittel einschliessen.

Die Verbindungen I und ihre Salze können, je nach Wahl der Ausgangsstoffe und Arbeitsweisen, in Form eines der möglichen Isomeren oder als Gemisch derselben vorliegen. Dabei sind als reine Isomere z.B. reine Diastereomere erhältlich. Entsprechend können als Isomerengemische z.B. Diastereomerengemische vorliegen. Verfahrensgemäss oder anderweitig erhältliche Isomerengemische von Verbindungen I in freier Form oder in Salzform können in üblicher Weise in die Komponenten aufgetrennt werden, z.B. aufgrund der physikalisch-chemischen Unterschiede der Bestandteile in bekannter Weise durch fraktionierte Kristallisation, Destillation und/oder Chromatographie. Vorteilhaft isoliert man das wirksamere Isomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes verwendet oder insbesondere unter den Reaktionsbedingungen bildet.

Beim Verfahren der vorliegenden Erfindung werden vorzugsweise solche Ausgangsstoffe und Zwischenprodukte, jeweils in freier Form oder in Salzform, verwendet, welche zu den eingangs als besonders wertvoll geschilderten Verbindungen I bzw. deren Salzen führen. Neue Ausgangsstoffe und Zwischenprodukte, jeweils in freier Form oder in Salzform, für die Herstellung der Verbindungen I bzw. ihrer Salze, ihre Verwendung und Verfahren zu ihrer Herstellung bilden ebenfalls einen Gegenstand der Erfindung, wobei die Variable R die für die Verbindungen I angegebene Bedeutung hat.

Die Erfindung betrifft ebenfalls die Verwendung der Verbindungen I und ihrer pharmazeutisch verwendbaren Salze zur Behandlung von allergischen Zuständen und Erkrankungen,vorzugsweise in Form von pharmazeutisch verwendbaren Zubereitungen, insbesondere in einem Verfahren zur therapeutischen Behandlung des tierischen oder menschlichen Körpers, sowie ein solches Behandlungsverfahren.

Die Erfindung betrifft gleichfalls pharmazeutische Präparate, die eine Verbindung I oder ein pharmazeutisch verwendbares Salz davon als Wirkstoff enthalten, sowie Verfahren zu ihrer Herstellung. Bei diesen pharmazeutischen Präparaten handelt es sich um solche zur enteralen, wie oralen, ferner rektalen, Verabreichung, um solche zur parenteralen Verabreichung, um solche zur lokalen Verabreichung und vor allem um solche zur Inhalationsverabreichung an Warmblüter, vor allem an Menschen, wobei der pharmakologische Wirkstoff allein oder zusammen mit üblichen pharmazeutischen Hilfsstoffen enthalten ist. Die pharmazeutischen Präparate enthalten (in Gewichtsprozenten) z.B. von etwa 0,001 % bis 100 %, vorzugsweise von etwa 0,1 % bis etwa 50 %, des Wirkstoffs.

Pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren, hergestellt.

So kann man pharmazeutische Präparate zur oralen Anwendung erhalten, indem man den Wirkstoff mit festen Trägerstoffen kombiniert, ein erhaltenes Gemisch gegebenenfalls granuliert und das Gemisch bzw. Granulat, wenn erwünscht oder notwendig nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragaganth, Methylcellulose und/oder Polyvinylpyrrolidon, und, wenn erwünscht, Sprengmittel, wie die obengenannten Stärken, ferner Carboxymethylstärke, quervernetztes Polyvinylpyrrolidon, Agar oder Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten, Ueberzügen versehen, wobei man u.a. konzentrierte Zuckerlösungen, welche gegebenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natriumcarboxymethylcellulose, Sorbit und/oder Dextran, und gegebenenfalls auch Stabilisatoren enthalten.

Pharmazeutische Präparate zur lokalen Verabreichung sind z.B. für die topische Behandlung der Haut Lotionen, Crèmes und Salben, d. h. flüssige oder semifeste Oel-in-Wasser- oder Wasser-in-Oel-Emulsionen, Fettsalben, die wasserfrei sind, Pasten, d. h. Crèmes und Salben mit sekretabsorbierenden Puderbestandteilen, Gele, die wässrig, wasserarm oder wasserfrei sind und aus quellbaren, gelbildenden Materialien bestehen, Schäume, d. h. in Aerosolform vorliegende flüssige Oel-in-Wasser-Emulsionen, welche aus Druckbehältern verabreicht werden, und eine wässrig-ethanolische Grundlage aufweisende Tinkturen, welche jeweils weitere übliche pharmazeutische Hilfsstoffe, wie Konservierungsmittel, enthalten können. Für die lokale Behandlung der Augen eignen sich z.B. Augentropfen, welche den Wirkstoff in steriler wässriger oder öliger Lösung enthalten, und Augensalben, die vorzugsweise ebenfalls in steriler Form hergestellt werden. Für die lokale Behandlung der Nase sind z.B. Sprays, ähnlich den weiter unten beschriebenen für die Behandlung der Atemwege, grobe Puder, die durch schnelles Inhalieren durch die Nasenlöcher verabreicht werden, und vor allem Nasentropfen, welche den Wirkstoff in wässriger oder öliger Lösung enthalten, geeignet. Für die lokale Behandlung der Mundhöhle eignen sich z.B. Lutschbonbons und Pastillen, welche den Wirkstoff in einer z. B. aus Zucker und Gummiarabikum oder Tragantgummi gebildeten inerten Masse enthalten, welcher Geschmacksstoffe beigegeben sein können. Die Herstellung der pharmazeutischen Präparate zur lokalen Verabreichung erfolgt in an sich bekannter Weise durch Vermischung des Wirkstoffs mit den pharmazeutischen Hilfsstoffen, z.B. durch Lösen oder Suspendieren des Wirkstoffs in der Grundlage oder in einem Teil davon, falls notwendig. Zur Herstellung von Emulsionen, in denen der Wirkstoff in einer der flüssigen Phasen gelöst ist, wird der Wirkstoff in der Regel vor der Emulgierung in dieser gelöst; zur Herstellung von Suspensionen, in denen der Wirkstoff in der Emulsion suspendiert ist, wird der Wirkstoff nach der Emulgierung mit einem Teil der Grundlage vermischt und dann dem Rest der Formulierung beigegeben.

Pharmazeutische Präparate zur Inhalationsverabreichung sind solche, in denen der Wirkstoff in mikronisierter Form vorliegt, d. h. in denen die Partikelgrösse des Wirkstoffs weniger als 20 µm, insbesondere weniger als 10 µm und vorteilhaft weniger als 5 µm, beträgt, z.B. mikronisierte Pulver und Aerosole, die in Form von Sprays verabreicht werden. Die mikronisierten Pulver enthalten den Wirkstoff allein oder zusammen mit einem inerten Trägerstoff, wie

Lactose, vorteilhaft zusammen mit einem der nachstehend erwähnten Treibmittel. Aerosole sind Lösungen, Suspensionen oder Emulsionen des Wirkstoffs in einer geeigneten, pharmazeutisch verwendbaren, flüssigen Phase, wie in Ethanol oder Wasser oder einem entsprechenden Gemisch, können nach Bedarf auch andere pharmazeutische Hilfsstoffe, wie nicht-ionische oder anionische oberflächenaktive Mittel, Emulgatoren und Stabilisatoren, und/oder Wirkstoffe anderer Art aufweisen und enthalten ein Treibmittel, z.B. ein inertes Gas, wie Butan, unter erhöhtem Druck oder insbesondere eine leicht flüchtige, vorzugsweise unter normalem Druck unterhalb der üblichen Raumtemperatur (z.B. zwischen etwa -30°C und etwa +10°C) siedende, Flüssigkeit, wie ein mindestens teilweise fluoriertes polyhalogeniertes Niederalkan, oder ein Gemisch solcher Flüssigkeiten. Zur Herstellung der pharmazeutischen Präparate in zur Inhalationsverabreichung fertiger Form wird eine entsprechende pharmazeutische Zusammensetzung zusammen mit dem Treibmittel in geeignete Behälter, wie Flacons oder Druckflaschen, abgefüllt, welche mit einer geeigneten Versprühungseinrichtung, z.B. einem Ventil, versehen sind. Das Ventil ist vorzugsweise als Dosierungsventil konstruiert, welches bei Betätigung eine vorbestimmte Menge des Behälterinhalts, entsprechend einer vorbestimmten Dosis des Wirkstoffs, freigibt. Bei der Herstellung der fertigen Arzneimittelform kann man auch so vorgehen, dass entsprechende Mengen der pharmazeutischen Zusammensetzung und des Treibmittels separat in die Behälter abgefüllt werden und erst dort zur Vermischung kommen.

Die Dosierung des Wirkstoffs kann von verschiedenen Faktoren, wie Wirksamkeit und Wirkungsdauer des Wirkstoffs, Stärke der zu behandelnden Krankheit bzw. ihrer Symptome, Applikationsweise, Warmblüter-Spezies, -Geschlecht, -Alter, -Gewicht und/oder individuellem Zustand des Warmblüters, abhängen. Im Normalfall ist für einen etwa 75 kg schweren Warmblüter eine, z.B. orale, Tagesdosis von etwa 1 mg bis etwa 150 mg, insbesondere von etwa 2,5 bis etwa 50 mg zu veranschlagen. Diese kann z.B. als Einmaldosis oder in mehreren Teildosen, z.B. solchen von 10 bis 50 mg, verabreicht werden.

Die nachfolgenden Beispiele illustrieren die vorstehend beschriebene Erfindung. Temperaturen sind in Grad Celsius angegeben. DMF steht für Dimethylformamid, Ethylacetat für Essigsäureethylester; Hexan meint ein Isomerengemisch verschiedener Hexane (Fa. Fluka).

Beispiel 1:

4-[3-(2-Chinolinylmethoxy)phenylaminol-2,2-diethyl-4-oxobutansäure

Eine Mischung von 0.87g 2,2-Diethylbernsteinsäure (H. Le Moal et al., Bull. Soc. Chim. 1964, 579-584) und 0.32ml Acetylchlorid in 10ml Dimethoxyethan wird während 2 Std. bei 85° gerührt und am Rotationsverdampfer eingedampft. Der Rückstand wird zweimal mit je 30ml Toluol behandelt und erneut eingedampft. Das erhaltene Anhydrid wird in 25ml Dimethoxyethan aufgenommen, mit 0.89g 3-(2-Chinolinylmethoxy)anilin [J.H. Musser et al., J. Med. Chem. 32 (1989) 1176-1183] und 1.44g Natriumacetat versetzt, 2 Std. bei 85° gerührt und eingedampft. Der Rückstand wird in 80ml 2N wässriger Salzsäure aufgeschlämmt und filtriert. Waschen des Niederschlags mit Wasser und Kristallisation aus Methanol ergibt die Titelverbindung als farblose Kristalle vom Smp. 149-149.5°. IR (Methylenchlorid): 3320, 3060, 2970, 2940, 2880, 2520, 1960, 1680, 1610, 1550, 1520, 1490, 1445, 1435, 1380, 1300, 1210, 1160, 1070, 970, 870, 830, 780, 750, 720, 690 $cm^{-1}$.

Beispiel 2: 4-[3-(7-Chlor-2-chinolinylmethoxy)phenylamino]-2,2-diethyl-4-oxobutansäure

Eine Mischung von 0.44g 2,2-Diethylbernsteinsäure und 1.6ml Acetylchlorid in 10ml Dimethoxyethan wird während 2 Std. bei 85° gerührt und am Rotationsverdampfer eingedampft. Der Rückstand wird zweimal mit je 30ml Toluol behandelt und erneut eingedampft. Das erhaltene Anhydrid wird in 20ml Dimethoxyethan aufgenommen, mit 0.51g 3-(7-Chlor-2-chinolinylmethoxy)anilin und 0.75g Natriumacetat versetzt, 5 Tage bei 20° gerührt und eingedampft. Der Rückstand wird in 80ml 2N wässriger Salzsäure aufgeschlämmt und filtriert. Waschen des Niederschlags mit Wasser und Kristallisation aus Methanol ergibt die Titelverbindung als farblose Kristalle vom Smp. 151-153°. IR(KBr): 3300, 2970, 2880, 1680, 1620, 1600, 1550, 1500, 1440, 1420, 1290, 1200, 1160, 1080, 940, 870, 850, 780, 680 $cm^{-1}$. DC (Hexan/Ethylacetat 1:1): $R_f$ = 0.17.

Das Ausgangsmaterial wird wie folgt hergestellt:

(a) 3-(7-Chlor-2-chinolinylmethoxy)anilin: Die Lösung von 1.02g 3-Aminophenol in 25ml abs. Methanol wird mit 1.8ml einer 5.4 m methanolischen Natriummethylatlösung versetzt, 10 Min. bei 20° gerührt und eingedampft. Der Rückstand wird in 20ml abs. DMF aufgenommen und bei 10° tropfenweise mit einer Lösung von 2.97g 2-Brommethyl-7-chlorchinolin [R. Zamboni, J. Med. Chem. 35 (1992) 3832-3844] in 10ml abs. DMF versetzt. Das Reaktionsgemisch wird 2 Std. bei 10° und 12 Std. bei 20° gerührt und dann eingedampft. Der Rückstand wird in Methylenchlorid aufgenommen, mit Wasser gewaschen, über Natriumsulfat getrocknet und am Rotationsverdampfer zur Trockene eingeengt.

Chromatographische Reinigung des Rückstandes an 200g Kieselgel mit Hexan/Ethylacetat 3:2 ergibt die Titelverbindung als oranges Oel, das kristallisiert; Smp. 87.5-88.5°.

Beispiel 3: 4-[3-(7-Fluor-2-chinolinylmethoxy)phenylamino]-2,2-diethyl-4-oxobutansäure

Eine Mischung von 2.14g 2,2-Diethylbernsteinsäure und 1.6ml Oxalylchlorid in 90ml Methylenchlorid wird während 5 Std. bei 30° gerührt und am Rotationsverdampfer eingedampft. Der Rückstand wird in 10ml Methylenchlorid aufgenommen und zu einer Lösung von 1.65g 3-(7-Fluor-2-chinolinylmethoxy)anilin in 45ml Methylenchlorid und 45ml Pyridin gegossen. Das Reaktionsgemisch wird 16 Std. bei 20° gerührt und eingedampft. Der Rückstand wird in 150ml 2N wässriger Salzsäure aufgeschlämmt und filtriert. Waschen des Niederschlags mit Wasser und Kristallisation aus Methanol ergibt die Titelverbindung als farblose Kristalle vom Smp. 164-165° (werden rötlich beim Schmelzen). IR (KBr): 3319, 2968, 1687, 1599, 1546, 1514, 1492, 1436, 1375, 1290, 1207, 1174, 1114, 1066, 965, 846, 774 $cm^{-1}$. DC (Methylenchlorid/Methanol 19:1): $R_f$= 0.25. Die Ausgangsmaterialien werden wie folgt hergestellt:

(a) 2-Brommethyl-7-fluorchinolin: Die Lösung von 13.03g 2-Methyl-7-fluorchinolin [Z. Song et al., J. Heterocyclic Chem. 30 (1993) 17-21] in 150ml Tetrachlorkohlenstoff wird mit 21.58g N-Bromsuccinimid und 0.21g Azoisobutyronitril versetzt. Die resultierende Suspension wird 27 Std. am Rückfluss gekocht, filtriert und eingedampft. Der Rückstand wird an Kieselgel mit Hexan/Ethylacetat 9:1 bis 7:3 chromatographiert. Die Titelverbindung wird in Form von farblosen Kristallen vom Smp. 101-102° erhalten.

(b) 3-(7-Fluor-2-chinolinylmethoxy)anilin: Die Lösung von 1.36g 3-Aminophenol in 150ml Aceton wird mit 1.99g Natriumcarbonat versetzt und 15 Min. bei 20° gerührt. Dieses Gemisch wird mit 3.0g 2-Brommethyl-7-fluorchinolin, 4.07g Cäsiumcarbonat und 0.1g Kaliumiodid versetzt und 3 Std. am Rückfluss gekocht. Das Reaktionsgemisch wird filtriert, der Niederschlag mit Aceton gewaschen und das Filtrat eingedampft. Der Rückstand wird in Methylenchlorid aufgenommen, mit Wasser gewaschen, über Natriumsulfat getrocknet und eingedampft. Das resultierende orange Oel wird an 200g Kieselgel mit Hexan/Ethylacetat 3:1 chromatographiert. Die Titelverbindung wird so als gelber Feststoff vom Smp. 88-89° erhalten.

Beispiel 4: 4-[3-(7-Trifluormethyl-2-chinolinylmethoxy)phenylamino]-2,2-diethyl-4-oxobutansäure

Eine Mischung von 0.52g 2,2-Diethylbernsteinsäure und 1.9ml Acetylchlorid in 10ml Dimethoxyethan wird während 1.5 Std. bei 85° gerührt und am Rotationsverdampfer eingedampft. Der Rückstand wird in 20ml Dimethoxyethan aufgenommen, mit 0.64g 3-(7-Trifluormethyl-2-chinolinylmethoxy)anilin und 1.21g Natriumacetat versetzt, 3 Std. bei 20° gerührt und eingedampft. Der Rückstand wird in 80ml 2N wässriger Salzsäure aufgeschlämmt und filtriert. Waschen des Niederschlags mit Wasser und Kristallisation aus Methanol ergibt die Titelverbindung als farblose Kristalle vom Smp. 173-175°. IR (KBr): 3318, 2971, 1709, 1667, 1600, 1536, 1495, 1429, 1321, 1299, 1262, 1188, 1127, 1056, 895, 862, 773, 683 $cm^{-1}$. DC (Hexan/Ethylacetat 1:1): $R_f$ = 0.30.

Die Ausgangsmaterialien werden wie folgt hergestellt:

(a) 2-Brommethyl-7-trifluormethylchinolin: Die Lösung von 14.45g 2-Methyl-7-trifluormethylchinolin [US-Patent 2 432 393 (Eastman Kodak Co., 1943)] in 150ml Tetrachlorkohlenstoff wird mit 18.26g N-Bromsuccinimid und 0.2g Azoisobutyronitril versetzt. Die resultierende Suspension wird 72 Std. am Rückfluss gekocht, filtriert und eingedampft. Der Rückstand wird an Kieselgel mit Hexan/Ethylacetat 3:2 chromatographiert. Die Titelverbindung wird als gelber Feststoff vom Smp. 71-72° erhalten.

(b) 3-(7-Trifluormethyl-2-chinolinylmethoxy)anilin: Die Lösung von 2.0g 3-Aminophenol in 200ml Aceton wird mit 3.0g Natriumcarbonat versetzt und 15 Min. bei 50° gerührt. Dieses Gemisch wird mit 5.2g 2-Brommethyl-7-trifluormethylchinolin, 6.1g Cäsiumcarbonat und 0.1g Kaliumiodid versetzt und 2 Std. am Rückfluss gekocht. Das Reaktionsgemisch wird filtriert, der Niederschlag mit Aceton gewaschen und das Filtrat eingedampft. Der Rückstand wird in Hexan/Ethylacetat 3:2 aufgenommen und über Kieselgel filtriert. Das Filtrat wird eingedampft und das resultierende Oel an 400g Kieselgel mit Hexan/Ethylacetat 3:2 chromatographiert. Die Titelverbindung wird als gelber Feststoff vom Smp. 85-86° erhalten.

Beispiel 5: 4-[7-(2-Chinolinylmethoxy)naphth-2-ylamino]-2,2-diethyl-4-oxobutansäure

Eine Mischung von 1.16g 2,2-Diethylbernsteinsäure und 4. lml Acetylchlorid wird während 2 Std. bei 52° gerührt und am Rotationsverdampfer eingedampft. Der Rückstand wird zweimal mit je 50ml Toluol behandelt und erneut eingedampft. Das erhaltene Anhydrid wird in 20ml Dimethoxyethan aufgenommen, mit 1.0g 7-(2-Chinolinylmethoxy)naphth-2-ylamin [US-Patent 4 719 308 (American Home Products, 1986)] und 1.37g Natriumacetat versetzt, 2 Std. bei 85° gerührt und eingedampft. Der Rückstand wird in 80ml 2N wässriger Salzsäure aufgeschlämmt und filtriert. Waschen des Niederschlags mit Wasser und Kristallisation aus Methanol ergibt die Titelverbindung als farblose Kristalle vom Smp. 190-191°.

Beispiel 6: 4-[7-(7-Fluor-2-chinolinylmethoxy)naphth-2-ylamino]-2,2-diethyl-4-oxo-butansäure

Eine Mischung von 0.67g 2,2-Diethylbernsteinsäure und 1.85ml Acetylchlorid wird während 2 Std. bei 52" gerührt und am Rotationsverdampfer eingedampft. De Rückstand wird zweimal mit je 20ml Toluol behandelt und erneut eingedampft. Das Anhydrid wird in 10ml Dimethoxyethan aufgenommen, mit 0.47g 7-(7-Fluor-2-chinolinylmethoxy) naphth-2-ylamin und 0.61g Natriumacetat versetzt, 2 Std. bei 85° gerührt und eingedampft. Der Rückstand wird in 40ml 2N wässriger Salzsäure aufgeschlämmt und filtriert. Waschen des Niederschlags mit Wasser und Kristallisation aus Methanol ergibt die Titelverbindung als farblose Kristalle vom Smp. 196-198°.

Das Ausgangsmaterial wird wie folgt hergestellt:

(a) 7-(7-Fluor-2-chinolinylmethoxy)naphth-2-ylamin: Zu einer Lösung von 0.13g Natrium in 10ml Methanol gibt man 0.86g 2-Amino-7-hydroxynaphthalin und rührt 1 Std. bei Raumtemperatur. Das Methanol wird im Vakuum abgedampft und der Rückstand in 13ml DMF gelöst. Dazu gibt man die Lösung von 1.3g 2-Brommethyl-7-fluorchinolin (Beispiel 3a) in 5ml DMF und rührt 6 Std. bei Raumtemperatur. Nach Zugabe von 100ml Wasser filtriert man den erhaltenen Niederschlag ab, kristallisiert aus Ethylacetat und erhält die Titelverbindung als hellgelbe Kristalle vom Smp. 164.5-165.5°.

Beispiel 7: 4-[3-(7-Fluor-2-chinolinylmethylthio)phenylamino]-2.2-diethyl-4-oxobutansäure

Eine Mischung von 0.95g 2,2-Diethylbernsteinsäure und 2.6ml Acetylchlorid wird während 2 Std. bei 52° gerührt und am Rotationsverdampfer eingedampft. Der Rückstand wird zweimal mit je 30ml Toluol behandelt und erneut eingedampft. Das erhaltene Anhydrid wird in 10ml Dimethoxyethan aufgenommen, mit 0.6g 3-(7-Fluor-2-chinolinylmethylthio)phenylamin [= 3-(7-Fluor-2-chinolinylmethylthio)anilin] und 0.86g Natriumacetat versetzt, 2 Std. bei 85° gerührt und eingedampft. Der Rückstand wird mit Methylenchlorid/Methanol 95:5 an Kieselgel chromatographiert. Zunächst erhält man einige Fraktionen, die geringe Mengen an Verunreinigungen enthalten; danach werden die das Produkt enthaltenden Fraktionen eluiert. Das Laufmittel wird unter vermindertem Druck abgedampft und der Rückstand aus Ethylacetat kristallisiert. Man erhält die Titelverbindung als beige Kristalle vom Smp. 132-134°.

Das Ausgangsmaterial wird wie folgt hergestellt:

(a) 3-(7-Fluor-2-chinolinylmethylthio)phenylamin: Zu einer Lösung von 0.13g Natrium in 10ml Methanol gibt man 0.86g 3-Aminothiophenol und rührt 1 Std. bei Raumtemperatur. Das Methanol wird im Vakuum abgedampft und der Rückstand in 13ml DMF gelöst. Dazu gibt man die Lösung von 1.3g 2-Brommethyl-7-fluorchinolin in 5ml DMF und rührt 6 Std. bei Raumtemperatur. Nach Zugabe von 100ml Wasser extrahiert man dreimal mit je 20ml Ethylacetat und trocknet die vereinigten Extrakte über Natriumsulfat. Nach Filtration und Eindampfen am Rotationsverdampfer wird der Rückstand aus Ether/n-Pentan 1:1 kristallisiert. Man erhält die Titelverbindung als hellgelbe Kristalle vom Smp. 70-71°.

Beispiel 8: 4-[3-(7-Fluor-2-chinolinylmethoxy)phenylaminol-2,2-diethyl-4-oxo-butansäuremethylester

Eine Lösung von 1.9g 4-[3-(7-Fluor-2-chinolinylmethoxy)phenylamino]-2,2-diethyl-4-oxobutansäure (Beispiel 3) in 150ml Tetrahydrofuran wird bei 0° solange mit einer Lösung von Diazomethan in Diethylether versetzt, bis eine Gelbfärbung bestehen bleibt. Die gelbe Lösung wird sofort eingedampft. Die Titelverbindung wird als farbloses Oel erhalten, das beim Stehen im Kühlschrank kristallisiert, Smp. 92-93°; IR (Methylenchlorid): 1735 $cm^{-1}$ (C=O).

Beispiel 9: Die nachfolgenden Verbindungen werden analog zu den in den Beispielen 1-8 beschriebenen Substanzen hergestellt:

(a) 4-[3-(6-Fluor-2-chinolinylmethoxy)phenylamino]-2,2-diethyl-4-oxobutansäure, Smp. 125-130°;

(b) 4-[3-(8-Fluor-2-chinolinylmethoxy)phenylamino]-2,2-diethyl-4-oxobutansäure, Smp. 150-152°;

(c) 4-[3-(6-Chlor-2-chinolinylmethoxy)phenylamino]-2,2-diethyl-4-oxobutansäure,

(d) 4-[3-(8-Chlor-2-chinolinylmethoxy)phenylamino]-2,2-diethyl-4-oxobutansäure,

(e) 4-[3-(7-Methyl-2-chinolinylmethoxy)phenylamino]-2,2-diethyl-4-oxobutansäure, Smp. 162-163°;

(f) 4-[3-(6-Methyl-2-chinolinylmethoxy)phenylamino]-2,2-diethyl-4-oxobutansäure, Smp. 175°;

(g) 4-[3-(8-Methyl-2-chinolinylmethoxy)phenylamino]-2,2-diethyl-4-oxobutansäure,

(h) 4-[3-(7-Methoxy-2-chinolinylmethoxy)phenylamino]-2,2-diethyl-4-oxobutansäure, Smp. 163-164°;

(i) 4-[3-(6-Methoxy-2-chinolinylmethoxy)phenylamino]-2,2-diethy 1-4-oxobutansäure,

(j) 4-[3-(8-Methoxy-2-chinolinylmethoxy)phenylamino]-2,2-diethyl-4-oxobutansäure,

(k) 4-[3-(7-Brom-2-chinolinylmethoxy)phenylamino]-2,2-diethyl-4-oxobutansäure, Smp. 175°;

(l) 4-[3-(7-Fluor-2-chinolinylmethylthio)phenylamino]-2,2- diethyl-4-oxobutansäure, Smp. 132-134°,

(m) 4-[3-(7-Chlor-2-chinolinylmethylthio)phenylamino]-2,2- diethyl-4-oxobutansäure,

(n) 4-[3-(2-Chinolinylmethylthio)phenylamino]-2,2-diethyl- 4-oxobutansäure,

(o) 4-[7-(7-Fluor-2-chinolinylmethoxy)naphth-2-ylamino]-2,2-di ethyl-4-oxobutansäure, Smp. 196-198°,

(p) 4-[7-(7-Chlor-2-chinolinylmethoxy)naphth-2-ylamino]-2,2-di ethyl-4-oxobutansäure,

(aa) 4-[7-(2-Chinolinylmethoxy)naphth-2-ylamino]-2,2-diethyl- 4-oxobutansäure, Smp. 190-191°,

(ab) 4-[N-(4-Carboxybenzyl)-3-(7-fluor-2-chinolinylmethoxy)phenylamino]-2,2-diethyl 4-oxobutansäuremethylester [1. N-Alkylierung von 3-(7-Fluor-2-chinolinylmethoxy) anilin, Beispiel 3b, mit 4-Carboxybenzylbromid; 2. Umsetzung des erhaltenen N-(4-Carboxybenzyl)-3-(7-fluor-2-chinolinylmethoxy)anilin mit 3-Ethyl-3-methoxy carbonyl-pentansäure (= Methylhalbester von 2,2-Diethylbernsteinsäure)],

(ac) 4-[N-(3-Carboxybenzyl)-3-(7-fluor-2-chinolinylmethoxy)phenylamino]-2,2-diethyl 4-oxobutansäuremethylester (aus N-(3-Carboxybenzyl)-3-(7-fluor-2-chinolinylmethoxy) anilin, analog zu Beispiel 9ab),

(ad) 4-[N-(2-Carboxybenzyl)-3-(7-fluor-2-chinolinylmethoxy)phenylamino]-2,2-diethyl 4-oxobutansäuremethylester (aus N-(2-Carboxybenzyl)-3-(7-fluor-2-chinolinylmethoxy) anilin, analog zu Beispiel 9ab),

(ae) 4-[3-(6,7-Dimethyl-2-chinolinylmethoxy)phenylamino]-2,2-diethyl-4-oxobutansäure [ausgehend von 2,6,7-Trimethylchinolin (Tetrahedron 39, 1983, 2831-2841) analog zu Beispiel 3], Smp. 184°.

(af) 4-[N-Methyl-3-(7-fluor-2-chinolinylmethoxy)phenylamino]-2,2-diethyl- 4-oxobutansäuremethylester (aus 4-[3-(7-Fluor-2-chinolinylmethoxy)phenylamino]- 2,2-diethyl--4-oxobutansäure, Beispiel 3, durch Umsetzung mit 2 Aequivalenten NaH und dann 2 Aequivalenten Methyliodid),

(ag) 4-[N-Methyl-3-(7-fluor-2-chinolinylmethoxy)phenylamino]-2,2-diethyl- 4-oxobutansäure (aus 4-[N-Methyl-3-(7-fluor-2-chinolinylmethoxy)phenylamino]- 2,2-diethyl-4-oxobutansäuremethylester, Beispiel 9af, (1) durch Verseifung mit LiOH/Methanol/Tetrahydrofuran/Wasser) oder (2) durch Reaktion mit 3 Aequivalenten Trimethylchlorsilan/Natriumiodid in Acetonitril bei 60°;

(ah) 4-[3-(7-Fluor-2-chinolinylmethoxy)phenylamino]-2,2-dimethyl-4-oxobutansäure (analog zu Beispiel 3 aus 2,2-Dimethylbernsteinsäure anstelle von 2,2-Diethylbern steinsäure), Smp. 209-210°;

(ai) 4-[3-(7-Fluor-2-chinolinylmethoxy)phenylamino]-2-ethyl-2- methyl-4-oxobutansäure (analog zu Beispiel 3 aus 2-Ethyl-2-methylbernsteinsäure anstelle von 2,2-Diethylbern steinsäure), Smp. 179-180°;

(aj) 1-[3-(7-Fluor-2-chinolinylmethoxy)phenylaminocarbonylmethyl]-cyclopentan carbonsäure [analog zu Beispiel 3 aus 2-(1-Carboxycyclopentyl)-essigsäure (Bull. Soc. Chim. Fr. 1964, 579-584) anstelle von 2,2-Diethylbernsteinsäure], Smp. 187-188°;

(ak) 1-[3-(7-Fluor-2-chinolinylmethoxy)phenylaminocarbonylmethyl]-cyclohexan carbonsäure [analog zu Beispiel 3 aus 2-(1-Carboxycyclohexyl)-essigsäure (Bull. Soc. Chim. Fr. 1964, 579-584) anstelle von 2,2-Diethylbern steinsäure], Smp. 190-191°;

(al) 4-[3-(7-Chlor-2-chinolinylmethoxy)phenylamino]-2,2-dimethyl- 4-oxobutansäure (analog zu Beispiel 2 aus 2,2-Dimethylbernsteinsäure anstelle von 2,2-Diethylbern steinsäure),

(am) 4-[3-(2-Chinolinylmethoxy)phenylamino]-2-ethyl-2-methyl- 4-oxobutansäure (analog zu Beispiel 1 aus 2-Ethyl-2-methylbernsteinsäure anstelle von 2,2-Diethylbernsteinsäure),

(an) 4-[3-(2-Chinolinylmethoxy)phenylamino]-2,2-di-n-propyl- 4-oxobutansäure (analog zu Beispiel 1 aus 2,2-Di-n-propylbernsteinsäure anstelle von 2,2-Diethylbernsteinsäure),

(ao) 4-[3-(2-Chinolinylmethoxy)phenylamino]-2,2-diethyl-4-oxobutansäure-methylester (aus der Verbindung des Beispiels 1 durch Veresterung z.B. mit Diazomethan),

(ap) 4-[3-(7-Chlor-2-chinolinylmethoxy)phenylamino]-2,2-diethyl-4-oxobutansäuremethylester (aus der Verbindung des Beispiels 2 durch Veresterung z.B. mit Diazomethan),

Beispiel 10: 4-[3-(6-Fluor-2-chinolinylmethoxy)phenylamino]-2,2-diethyl-4-oxobutansäure

Eine Mischung von 0.34g 2,2-Diethylbernsteinsäure und 0.2ml Acetylchlorid wird während 2 Std. bei 52° gerührt und am Rotationsverdampfer eingedampft. Der Rückstand wird zweimal mit je 20ml Toluol behandelt und erneut eingedampft. Das erhaltene 2,2-Diethylbernsteinsäureanhydrid wird in 3ml Dichlormethan gelöst, mit 0.2g 3-(6-Fluor-2-chinolinylmethoxy)anilin und mit 3ml Pyridin versetzt, 4 Std. bei Raumtemperatur gerührt und mit 100ml Wasser hydrolysiert. Die organische Phase wird im Scheidetrichter abgetrennt und die wässrige Phase zweimal mit je 10ml Dichlormethan extrahiert. Nach Trocknen über Natriumsulfat und Abdampfen des Lösungsmittels i.Vak. wird der Rückstand aus Methanol umkristallisiert. Man erhält die Titelverbindung als farblose Kristalle vom Smp. 125-130°.

Das Ausgangsmaterial wird wie folgt hergestellt:

(a) 2-Brommethyl-6-fluorchinolin: Die Lösung von 7.0g 2-Methyl-6-fluorchinolin [Song et al., J. Heterocylic Chem. 30 (1993) 17-21] in 60ml Tetrachlormethan wird mit 11.5g N-Bromsuccinimid und 0.1g Azobisisobutyronitril versetzt, 7 Std. am Rückfluss erhitzt, filtriert und eingedampft. Der Rückstand wird an Kieselgel mit Hexan/Ethylacetat 3:2 chromatographiert. Man erhält die Titelverbindung als gelbe Kristalle vom Smp. 88-90°.

(b) 3-(6-Fluor-2-chinolinylmethoxy)anilin: Zu einer Lösung von 0.3g Natrium in 15ml Methanol gibt man 1.74g 3-Aminophenol und rührt 1 Std. bei Raumtemperatur. Das Methanol wird i.Vak. abgedampft und der Rückstand in 30ml Dimethylformamid gelöst. Dazu gibt man die Lösung von 3.0g 2-Brommethyl-6-fluorchinolin in 12ml Dimethylformamid und rührt 12 Std. bei Raumtemperatur. Das Lösungsmittel wird i.Vak. abgedampft und der Rückstand mit 100ml Wasser versetzt, mit Ethylacetat (zweimal je 15ml) extrahiert und über Natriumsulfat getrocknet. Nach Filtration und Abdampfen des Lösungsmittels i.Vak. wird der Rückstand mit Hexan/Ethylacetat 1:1 an Kieselgel chromatographiert. Man erhält die Titelverbindung als hellgelbes Oel. $^1$H-NMR (300 MHz, $CDCl_3$): delta = 5.33 (s, 2H: $CH_2$), 6.20-6.39 (m, 2H), 6.43 (m, 1H), 7.04 (m, 1H), 7.42-7.56 (m, 2H), 7.69 (d, 1H), 8.07 (m, 1H), 8.14 (d, 1H) ppm.

Beispiel 11: 4-[7-(6-Fluor-2-chinolinylmethoxy)naphth-2-ylamino]-2,2-diethyl-4-oxobutansäure

Analog Beispiel 6 wird die Titelverbindung ausgehend von 1.3g 2,2-Diethylbernsteinsäure und 0.92g 7-(6-Fluor-2-chinolinylmethoxy)naphth-2-ylamin als beige Kristalle vom Smp. 191-196° erhalten.

Das Ausgangsmaterial wird wie folgt hergestellt:
(a) 7-(6-Fluor-2-chinolinylmethoxy)naphth-2-ylamin: Ausgehend von 5.3g 2-Amino-7-hydroxynaphthalin und 8.0g 2-Brommethyl-6-fluorchinolin (Beispiel 10a) erhält man die Titelverbindung analog Beispiel 6(a); $^1$H-NMR (300 MHz, $CDCl_3$): delta = 5.45 (s, 2H: $CH_2$), 6.80 (dd, 1H), 6.85 (d, 1H), 7.10 (d, 1H), 7.40 (dd, 1H), 7.45 (dd, 1H), 7.52 (dt, 1H), 7.58 (d, 1H), 7.63 (d, 1H), 7.73 (d, 1H), 8.10 (dd, 1H), 8.14 (d, 1H) ppm.

Beispiel 12: 4-[3-(6-Fluor-2-chinolinylmethylthio)phenylaminol-2,2-diethyl-4-oxobutansäure

Analog Beispiel 7 wird die Titelverbindung ausgehend von 4.48g 2,2-Diethylbernsteinsäure und 2.40g 3-(6-Fluor-2-chinolinylmethylthio)anilin als beige Kristalle vom Smp. 117-120° erhalten.

Das Ausgangsmaterial wird wie folgt hergestellt:

(a) 3-(6-Fluor-2-chinolinylmethylthio)anilin: Ausgehend von 3.49g 3-Aminothiophenol und 6.70g 2-Brommethyl-6-fluorchinolin [Beispiel 10(a)] erhält man die Titelverbindung als Kristalle vom Smp. 69-73° analog Beispiel 7(a).

Beispiel 13: 4-[3-(8-Fluor-2-chinolinylmethoxy)phenylamino]-2,2-diethyl-4-oxobutansäure

Analog Beispiel 10 wird die Titelverbindung ausgehend von 4.68g 2,2-Diethylbernsteinsäure und 2.80g 3-(8-Fluor-2-chinolinylmethoxy)anilin als beige Kristalle vom Smp. 150-152° erhalten.

Das Ausgangsmaterial wird wie folgt hergestellt:

(a) 2-Methyl-8-fluorchinolin: 45.6g 2-Fluoranilin werden in 450ml 2-Butanol vorgelegt, mit 140ml 6.6molarer Lösung von HCl in 2-Butanol und 155.6g Chloranil versetzt. Unter Rühren wird die Mischung zum Rückfluss erhitzt. Dann tropft man die Lösung von 40.0g Crotonaldehyd in 120ml 2-Butanol zum Reaktionsgemisch und erhitzt 50 Min. am Rückfluss. Nach dem Abkühlen wird das Lösungsmittel i.Vak. weitgehend abgedampft, 700 ml Tetrahydrofuran zugesetzt und 30 Min. am Rückfluss erhitzt. Nach dem Abkühlen wird der Niederschlag abfiltriert und zwischen 1 Liter 2N wässriger Natronlauge und 400ml Dichlormethan verteilt. Die organische Phase wird abgetrennt und die wässrige Phase noch zweimal mit je 200ml Dichlormethan extrahiert. Nach Trocknen über Natriumsulfat, Filtration und Abdampfen des Lösungsmittels i.Vak. sowie Chromatographie des Rückstandes mit Dichlormethan an Kieselgel erhält man die Titelverbindung; $^1$H-NMR (300 MHz, $CDCl_3$): delta = 2.80 (s, 3H, $CH_3$), 7.31-7.44 (m, 3H), 7.55 (m, 1H), 8.05 (dd, 1H) ppm.

(b) 2-Brommethyl-8-fluorchinolin: Analog Beispiel 10(a) erhält man die Titelverbindung ausgehend von 10g 2-Methyl-8-fluorchinolin und 16.5g N-Bromsuccinimid als hellgelbe Kristalle vom Smp. 89-93°.

(c) 3-(8-Fluor-2-chinolinylmethoxy)nitrobenzol: 0.67g 3-Nitrophenol und 1.15g 2-Brommethyl-8-fluorchinolin werden in 110ml Ethylmethylketon gelöst, mit 3.3g Kaliumcarbonat und 0.8g Kaliumiodid versetzt und unter Rühren 3 Std. am Rückfluss erhitzt. Nach dem Abkühlen, Abfiltrieren des Feststoffes und Abdampfen des Lösungsmittels i.Vak. wird der Rückstand mit 100ml Dichlormethan versetzt, mit Wasser (zweimal je 20ml) gewaschen, über Natriumsulfat getrocknet, filtriert und i.Vak. vom Lösungsmittel befreit. Man erhält die Titelverbindung nach Umkristallisieren aus Dichlormethan/Hexan als hellgelbe Kristalle vom Smp. 128-129°.

(d) 3-(8-Fluor-2-chinolinylmethoxy)anilin: 1.2g 3-(8-Fluor-2-chinolinylmethoxy)nitrobenzol werden in 40ml Tetrahydrofuran gelöst, mit 3.5g Raney-Nickel versetzt und bei Raumtemperatur unter Normaldruck hydriert. Nach Abfiltrieren des Katalysators und Abdampfen des Lösungsmittels i.Vak. wird der Rückstand mit Dichlormethan/Hexan/Ethylacetat 6:3:1 an Kieselgel chromatographiert. Man erhält die Titelverbindung als hellgelbe Kristalle vom Smp. 94-97°.

Beispiel 14: 4-[7-(8-Fluor-2-chinolinylmethoxy)naphth-2-ylamino]-2,2-diethyl-4-oxobutansäure

Analog Beispiel 6 wird die Titelverbindung ausgehend von 0.41g 2,2-Diethylbernsteinsäure und 0.29g 7-(8-Fluor-2-chinolinylmethoxy)naphth-2-ylamin als beige Kristalle vom Smp. 193-196° erhalten.

Das Ausgangsmaterial wird wie folgt hergestellt:

(a) 7 - (8 -Fluor -2 -chinolinylmethoxy )naphth -2 -ylamin: Ausgehend von 3.3g 2-Amino-7-hydroxynaphthalin und 5.0g 2-Brommethyl-8-fluorchinolin [Beispiel 13(b)] erhält man die Titelverbindung als beige Kristalle vom Smp. 141-142° analog Beispiel 6(a).

Beispiel 15: 4-[3-(8-Fluor-2-chinolinylmethylthio)phenylamino]-2,2-diethyl-4-oxobutansäure

Analog Beispiel 7 wird die Titelverbindung ausgehend von 4.97g 2,2-Diethylbernsteinsäure und 3.15g 3-(8-Fluor-2-chinolinylmethylthio)anilin als beige Kristalle vom Smp. 113-115° erhalten.

Das Ausgangsmaterial wird wie folgt hergestellt:

(a) 3-(8-Fluor-2-chinolinylmethylthio)anilin: Ausgehend von 2.72g 3-Aminothiophenol und 5.20g 2-Brommethyl-8-fluorchinolin [Beispiel 13(b)] erhält man die Titelverbindung als Kristalle vom Smp. 80-83° analog Beispiel 7(a).

Beispiel 16: 4-[3-(6,7-Difluor-2-chinolinylmethoxy)phenylamino]-2,2-diethyl-4-oxobutansäure

Analog Beispiel 3 wird die Titelverbindung aus 2,2-Diethylbernsteinsäure und 3-(6,7-Difluor-2-chinolinylmethoxy)anilin hergestellt. Sie wird als weisser Feststoff erhalten; $^1$H-NMR, 200MHz, DMSO, delta (ppm) = 9.90(s, 1H), 8.42(d, 1H), 8.10(m, 2H), 7.70(d, 1H), 7.45(m, 1H), 7.07(m, 2H), 6.73(d, 1H), 5.35(s, 2H), 2.58(s, 2H), 1.80(m, 4H), 0.80(t, 6H).

Die Ausgangsmaterialien werden wie folgt hergestellt:

(a) 2-Methyl-6,7-difluorchinolin: Ausgehend von 3,4-Difluoranilin wird die Titelverbindung analog Beispiel 13(a) durch Umsetzung mit Crotonaldehyd und Chloranil in 2-Butanol hergestellt [vgl. auch J. Heterocycl. Chem. 30 (1993) 17-21].

(b) 3-(6,7-Difluor-2-chinolinylmethoxy)anilin: Ausgehend von 2-Methyl-6,7-difluorchinolin wird die Titelverbindung analog Beispiel 3(a) [NBS-Bromierung] und Beispiel 3(b) [Umsetzung mit 3-Aminophenol] hergestellt: $^1$H-NMR, 200MHz, DMSO, delta (ppm) = 8.42(d, 1H), 8.10(m, 2H), 7.67(d, 1H), 7.45(m, 1H), 6.90(t, 1H), 6.20(m, 3H), 5.25 (s, 2H), 5.08(s, 2H).

Beispiel 17: 4-[N-(4-Carboxy-2-methoxybenzyl)-3-(7-fluor-2-chinolinylmethoxy)phenyl]amino]-2,2-diethyl-4-oxobutansäure

0.12g 4-[N-(4-Methoxycarbonyl-2-methoxybenzyl)-3-(7-fluor-2-chinolinylmethoxy)phenyl]amino]-2,2-diethyl-4-oxobutansäuremethylester (Beispiel 17A) und 2ml 10N Natronlauge werden für 20Std. in Wasser/Methano/Tetrahydrofuran 1:1:1 am Rückfluss gekocht. Anschliessend wird durch Zugabe von von 1N HCl auf einen pH-Wert von etwa 2 gestellt und mit Methylenchlorid/Isopropanol 4:1 dreimal extrahiert. Die vereinigten organischen Phasen werden mit gesättigter Kochsalzlösung gewaschen, getrocknet (Magnesiumsulfat) und eingedampft. Der Rückstand wird in Diethylether aufgerührt, abfiltriert und getrocknet. Auf diese Weise wird die Titelverbindung in Form eines weissen amorphen Feststoffes erhalten; $^1$H-NMR, 400MHz, DMSO, delta (ppm) = 8.40(d, 2H), 8.05(dd, 1H), 7.67(dd, 1H), 7.58(d, 1H), 7.2-7.5(m, 4H), 7.0(m, 1H), 6.86(m, 1H), 6.75(m, 1H), 5.37(s, 2H), 4.80(s, 2H), 3.68(s, 3H), 2.30(s, 2H), 1.50(q, 4H), 0.6(t, 6H).

Beispiel 17A: 4-[N-(4-Methoxycarbonyl-2-methoxybenzyl)-3-(7-fluor-2-chinolinylmethoxy)phenyl]amino]2,2-diethyl-4-oxobutansäuremethylester

Zu einer Lösung von 0.22g N-(4-Methoxycarbonyl-2-methoxybenzyl)-3-(7-fluor-2-chinolinylmethoxy)anilin in 20ml Methylenchlorid werden 0.1g 3-Ethyl-3-methoxycarbonyl-pentansäure (= Methylhalbester von 2,2-Diethylbernsteinsäure; vgl. Bull. Soc. Chim. 1964, 828), 0.38g N-Ethyl-N-(3-dimethylaminopropyl)-carbodiimid-hydrochlorid und 0.24g 4-Dimethylamino-pyridin gegeben. Das Reaktionsgemisch wird unter Argon 72Std. bei Raumtemperatur gerührt. Anschliessend wird das Reaktionsgemisch mit 100ml Ethylacetat versetzt, und die entstandene trübe Lösung wird dreimal mit Wasser und einmal mit gesättigter Kochsalzlösung gewaschen, getrocknet (Magnesiumsulfat) und eingedampft. Der Rückstand wird an Kieselgel (Hexan/Ethylacetat 3:2) gereinigt. Auf diese Weise wird die Titelverbindung in Form eines Oels erhalten; $^1$H-NMR, 200MHz, $CDCl_3$, delta (ppm) = 8.17(d, 1H), 7.83(dd, 1H), 7.70(dd, 1H), 7.55(d, 2H), 7.40-7.18(m, 4H), 6.95(dd, 1H), 6.65(m, 2H), 5.30(s, 2H), 4.90(s, 2H), 3.90(s, 3H), 3.70(2s, 6H), 2.37(s, 2H), 1.60 (q, 4H), 0.65(t, 6H).

Das Ausgangsmaterial wird wie folgt hergestellt:
(a) N-(4-Methoxycarbonyl-2-methoxybenzyl)-3-(7-fluor-2-chinolinylmethoxy)anilin: Eine Mischung bestehend aus 0.536g 3-(7-Fluor-2-chinolinylmethoxy)anilin, 0.518g 3-Methoxy-4-brommethyl-benzoesäuremethylester, 1.0g Natriumcarbonat und 0.2g Kaliumiodid in 50ml Ethylmethylketon wird für 18Std. unter Argon am Rückfluss gekocht. Anschliessend wird das Reaktiongemisch filtriert und eingedampft. Der Rückstand wird an Kieselgel (Hexan/Ethylacetat 3:2) gereinigt. Auf diese Weise wird die Titelverbindung in Form eines Oels erhalten; $^1$H-NMR, 200MHz, $CDCl_3$, delta (ppm) = 8.12(d, 1H), 7.80(dd, 1H), 7.5-7.7(m, 4H), 7.25-7.35(m, 2H), 7.05(t, 1H), 5.30(s, 2H), 4.35(s, 2H), 3.90(2s, 6H).

Beispiel 18: 4-[3-(7-Brom-2-chinolinylmethoxy)phenylamino]-2,2-diethyl-4-oxobutansäure

2g 3-(7-Brom-2-chinolinylmethoxy)anilin und 1,9g 2,2-Diethylbernsteinsäureanhydrid (durch Destillation gereinigt) werden in 28ml Pyridin 72Std. bei 20° gerührt und eingedampft. Der Rückstand wird in 25ml 2N wässriger Salzsäure aufgeschlämmt, 30Min. bei 20° gerührt und abfiltriert. Waschen des Niederschlags mit Wasser und Kristallisation aus Ethanol ergibt die Titelverbindung als farblose Kristalle vom Smp.175°, IR (Nujol): u.a. 3600-2100, 3308, 1710, 1670, 1610, 1600, 1497cm$^{-1}$.

Das Ausgangsmaterial wird wie folgt hergestellt:

(a) 2-Methyl-7-brom-chinolin-1-oxid: Zu einer Lösung von 7,09g 2-Methyl-7-brom- chinolin [C.M.Leir, J.Org. Chem. 42(5) (1977) 911-13] in 75ml n-Hexan tropft man bei 0-10° im Verlaufe von 30Min. eine Lösung von 10,05g m-

Chlorperbenzoesäure in 75ml $CH_2Cl_2$ zu und rührt anschliessend 16Std. bei 20°. Dann verdünnt man mit Ethylacetat, wäscht nacheinander mit 2N wässrigen Lösungen von $K_2CO_3$, $Na_2S_2O_3$ sowie NaCl, trocknet über $Na_2SO_4$ und dampft ein. Dabei erhält man die Titelverbindung, die durch Kristallisation aus $CH_2Cl_2$-Ether-Hexan gereinigt wird.

(b) 2-Chlormethyl-7-brom-chinolin: Zu einer Lösung von 5g 2-Methyl-7-brom- chinolin-1-oxid in 32ml Toluol tropft man unter Rühren bei 50° im Verlaufe von einer Stunde eine Lösung von 5,7ml Benzolsulfochlorid in 6,5ml Toluol zu und rührt 16Std. bei 50° nach. Dann nimmt man in Ethylacetat auf, wäscht mit 2N wässrigen Lösungen von $NaHCO_3$ sowie NaCl, trocknet über $Na_2SO_4$ und dampft ein. Der Eindampfrückstand wird an Kieselgel chromatographiert. Die produkthaltigen, mit Toluol eluierten Fraktionen werden vereinigt und eingedampft. Dabei erhält man die Titelverbindung, die durch Kristallisation aus $CH_2Cl_2$-Ether-Hexan weiter gereinigt wird.

(c) 3-(7-Brom-2-chinolinylmethoxy)anilin: Eine Lösung von 1,38g 3-Aminophenol in 30ml DMF versetzt man bei 0° mit 0,32g NaH (95%) und rührt 30Min. bei 0° nach. Dann gibt man 3,2g 2-Chlormethyl-7-brom-chinolin in fester Form dazu und rührt eine weitere Stunde bei 0° sowie nochmals eine Stunde bei 20°. Anschliessend wird mit Ethylacetat verdünnt, mit Wasser gewaschen, über $Na_2SO_4$ getrocknet und eingedampft. Der Eindampfrückstand wird an 250g Kieselgel chromatographiert. Die produkthaltigen, mit Toluol/Ethylacetat 95:5 eluierten Fraktionen werden vereinigt und eingedampft. Dabei erhält man die Titelverbindung, die durch Kristallisation aus $CH_2Cl_2$-Ether-Hexan weiter gereinigt wird.

Beispiel 19: 4-[3-(6,7-Cyclopentano-2-chinolinylmethoxy)phenylamino]-2,2-diethyl-4-oxobutansäure

2,48g 3-(6,7-Cyclopentano-2-chinolinylmethoxy)anilin und 2,67g 2,2-Diethylbernstein säureanhydrid werden in 40ml Pyridin 72Std. bei 20° gerührt und eingedampft. Der Rückstand wird in 25ml 2N wässriger Salzsäure aufgeschlämmt, 30Min. bei 20° gerührt und abfiltriert. Waschen des Niederschlags mit Wasser und Kristallisation aus Ethanol ergibt die Titelverbindung als farblose Kristalle vom Smp.180°, IR (KBr): u.a. 3600-2200, 3325, 1683, 1600, 1542$cm^{-1}$.

Das Ausgangsmaterial wird wie folgt hergestellt:

(a) 2-Methyl-6,7-cyclopentano-chinolin-1-oxid: Zu einer Lösung von 11,6g 2-Methyl-6,7-cyclopentano-chinolin [Lindner et al. Monatsh. Chem. 72 (1939) 354, 356] in 120ml $CH_2Cl_2$ tropft man bei 0-10° im Verlaufe von 30Min. eine Lösung von 19,9g m-Chlorperbenzoesäure in 120ml $CH_2Cl_2$ zu und rührt anschliessend 16Std. bei 20°. Dann verdünnt man mit Ethylacetat, wäscht nacheinander mit 2N wässrigen Lösungen von $K_2CO_3$, $Na_2S_2O_3$ sowie NaCl, trocknet über $Na_2SO_4$ und dampft ein. Dabei erhält man die Titelverbindung, die durch Kristallisation aus $CH_2Cl_2$-Ether-Hexan gereinigt wird.

(b) 2-Chlormethyl-6,7-cyclopentano-chinolin: Zu einer Lösung von 7,32g 2-Methyl-6,7-cyclopentano-chinolin-1-oxid in 100ml Toluol tropft man unter Rühren bei 50° im Verlaufe von einer Stunde eine Lösung von 9,47ml Benzolsulfochlorid in 12ml Toluol zu und rührt 16Std. bei 50° nach. Dann nimmt man in Ethylacetat auf, wäscht mit 2N wässrigen Lösungen von $NaHCO_3$ sowie NaCl, trocknet über $Na_2SO_4$ und dampft ein. Der Eindampfrückstand wird an 250g Kieselgel chromatographiert. Die produkthaltigen, mit Toluol und Toluol/Essigester 98:2 eluierten Fraktionen werden vereinigt und eingedampft. Dabei erhält man die Titelverbindung, die durch Kristallisation aus $CH_2Cl_2$-Ether-Hexan weiter gereinigt wird.

(c) 3-(6,7-Cyclopentano-2-chinolinylmethoxy)anilin: Eine Lösung von 1,35g 3-Aminophenol in 30ml DMF versetzt man bei 0° mit 0,313g NaH (95%) und rührt 30Min. bei 0° nach. Dann gibt man 2,66g 2-Chlormethyl-6,7-cyclopentano-chinolin in fester Form dazu und rührt eine weitere Stunde bei 0° sowie nochmals eine Stunde bei 20°. Anschliessend wird mit Ethylacetat verdünnt, mit Wasser gewaschen, über $Na_2SO_4$ getrocknet und eingedampft. Der Eindampfrückstand wird an 100g Kieselgel chromato graphiert. Die produkthaltigen, mit Toluol/Ethylacetat 95: 5 und 9:1 eluierten Fraktionen werden vereinigt und eingedampft. Dabei erhält man die Titelverbindung, die durch Kristallisation aus $CH_2Cl_2$-Ether-Hexan weiter gereinigt wird.

Beispiel 20: 4-[3-(7-Trifluormethoxy-2-chinolinylmethoxy)phenylamino]-2,2-diethyl-4-oxobutansäure

1,625g 3-(7-Trifluormethoxy-2-chinolinylmethoxy)anilin und 1,52g 2,2-Diethylbernsteinsäureanhydrid werden in 26ml Pyridin 72Std. bei 20° gerührt und eingedampft. Der Rückstand wird in 16ml 2N wässriger Salzsäure aufgeschlämmt, 30Min. bei 20° gerührt und abfiltriert. Waschen des Niederschlags mit Wasser und Kristallisation aus Ethanol ergibt die Titelverbindung als Kristalle vom Smp.172°, IR (KBr): u.a. 3620-2500, 3330, 1690, 1595, 1545$cm^{-1}$.

Das Ausgangsmaterial wird wie folgt hergestellt:

(a) 2-Methyl-7-trifluormethoxy-chinolin: 4,85g 3-Trifluormethoxyanilin löst man in 31,5ml 2-Butanol, fügt 11ml 4,8N HCl in 2-Butanol sowie 6,22g 2,3-Dichlor- 1,4-naphthochinon zu und erwärmt auf Rückflusstemperatur. Anschliessend tropft man eine Lösung von 2,74ml Crotonaldehyd in 6,5ml 2-Butanol im Verlaufe von 50Min. zu und rührt bei Rückflusstemperatur 20Min. nach. Dann dampft man ein, fügt nochmals 35ml 2-Butanol zu und wiederholt den Eindampfvorgang. Durch zweimalige Umkristallisation des Eindampfrückstandes aus Methanol-Tetrahydrofuran erhält man das Hydrochlorid der Titelverbindung in gereinigter Form. Zur Freisetzung der Titelverbindung wird das obige Hydrochlorid in Wasser suspendiert, mit 1N wässriger NaOH basisch gestellt und die Wasserphase mit Ethylacetat extrahiert. Die Essigesterphase wird mit gesättigter wässriger NaCl-Lösung neutral gewaschen, über $Na_2SO_4$ getrocknet und eingedampft. Die dabei in Form des Eindampfrückstandes anfallende Titelverbindung wird ohne Reinigung in die nächste Stufe eingesetzt.

(b) 2-Methyl-7-trifluormethoxy-chinolin-1-oxid: Zu einer Lösung von 3g 2-Methyl-7-trifluormethoxy-chinolin in 30ml $CH_2Cl_2$ tropft man bei 0-10° unter Rühren im Verlaufe von 30Min. eine Lösung von 4,15g m-Chlorperbenzoesäure in 30ml $CH_2Cl_2$ zu und rührt anschliessend 16Std. bei 20°. Dann verdünnt man mit Ethylacetat, wäscht nacheinander mit 2N wässrigen Lösungen von $K_2CO_3$, $Na_2S_2O_3$ sowie NaCl, trocknet über $Na_2SO_4$ und dampft ein. Dabei erhält man die Titelverbindung, die durch Kristallisation aus $CH_2Cl_2$-Ether gereinigt wird.

(c) 2-Chlormethyl-7-trifluormethoxy-chinolin: Zu einer Lösung von 2,53g 2-Methyl-7-trifluormethoxy-chinolin-1-oxid in 35ml Toluol tropft man unter Rühren bei 50° im Verlaufe von einer Stunde 2,68ml Benzolsulfochlorid in 3,5ml Toluol zu und rührt 16Std. bei 50° nach. Dann nimmt man in Ethylacetat auf, wäscht mit 2N wässrigen Lösungen von $NaHCO_3$ sowie NaCl, trocknet über $Na_2SO_4$ und dampft ein. Der Eindampfrückstand wird an Kieselgel (100g) chromatographiert. Die produkthaltigen, mit Toluol eluierten Fraktionen werden vereinigt und eingedampft. Dabei erhält man die Titelverbindung, die direkt weiterverarbeitet wird.

(d) 3-(7-Trifluormethoxy-2-chinolinylmethoxy)anilin: Eine Lösung von 0,754g 3-Aminophenol in 20ml DMF versetzt man bei 0° mit 0,175g NaH (95%) und rührt 30Min. bei 0° nach. Dann gibt man 1,78g 2-Chlormethyl-7-trifluormethoxy-chinolin in fester Form dazu und rührt eine weitere Stunde bei 0° sowie nochmals eine Stunde bei 20°. Anschliessend wird mit Ethylacetat verdünnt, mit Wasser gewaschen, über $Na_2SO_4$ getrocknet und eingedampft. Der Eindampfrückstand wird an 100g Kieselgel chromato graphiert. Die produkthaltigen, mit Toluol/Ethylacetat 98:2 und 95:5 eluierten Fraktionen werden vereinigt und eingedampft. Dabei erhält man die Titelverbindung, die durch Kristallisation aus $CH_2Cl_2$-Ether-Hexan weiter gereinigt wird.

Beispiel 21: 4-[3-(6-Methyl-2-chinolinylmethoxy)phenylamino]-2,2-diethyl-4-oxobutansäure

2,05g 3-(6-Methyl-2-chinolinylmethoxy)anilin und 2,42g 2,2-Diethylbernsteinsäure anhydrid werden in 30ml Pyridin 72Std. bei 20° gerührt und eingedampft. Der Rückstand wird in 25ml 2N wässriger Salzsäure aufgeschlämmt, 30Min. bei 20° gerührt und abfiltriert. Waschen des Niederschlags mit Wasser und Kristallisation aus Ethanol ergibt die Titelverbindung als Kristalle vom Smp.175°, IR (Nujol): u.a. 3600-2600, 3320, 1685, 1545cm$^{-1}$.

Das Ausgangsmaterial wird wie folgt hergestellt:

(a) 3-(6-Methyl-2-chinolinylmethoxy)anilin: Eine Lösung von 1,85g 3-Aminophenol in 30ml DMF versetzt man bei 0° mit 0,43g NaH (95%) und rührt 30Min. bei 0° nach. Dann gibt man 3,2g 2-Chlormethyl-6-methyl-chinolin [s. C.A. 82, 57733p und US-Patent 3829573] in fester Form dazu und rührt eine weitere Stunde bei 0° sowie nochmals eine Stunde bei 20°. Anschliessend wird mit Ethylacetat verdünnt, mit Wasser gewaschen, über $Na_2SO_4$ getrocknet und eingedampft. Der Eindampfrückstand wird an 250g Kieselgel chromatographiert. Die produkthaltigen, mit Toluol/Ethylacetat 95:5 und 9:1 eluierten Fraktionen werden vereinigt und eingedampft. Dabei erhält man die Titelverbindung, die durch zweimalige Kristallisation aus $CH_2Cl_2$-Ether-Hexan weiter gereinigt wird.

Beispiel 22: 4-[3-(6,7-Dimethyl-2-chinolinylmethoxy)phenylamino]-2'2-diethyl-4-oxobutansäure

1,65g 3-(6,7-Dimethyl-2-chinolinylmethoxy)anilin und 1,85g 2,2-Diethylbernsteinsäureanhydrid werden in 25ml Pyridin 72Std. bei 20° gerührt und eingedampft. Der Rückstand wird in 25ml 2N wässriger Salzsäure aufgeschlämmt, 30Min. bei 20° gerührt und abfiltriert. Waschen des Niederschlags mit Wasser und Kristallisation aus Ethanol ergibt die Titelverbindung als Kristalle vom Smp.184°, IR (KBr): u.a. 3600-2500, 3320, 1680, 1600, 1547cm$^{-1}$.

Das Ausgangsmaterial wird wie folgt hergestellt:

(a) 2,6,7-Trimethyl-chinolin-1-oxid: Zu einer Lösung von 8,6g 2,6,7-Trimethyl-chinolin [A.G.Osborn, Tetrahedron 39(17) (1983) 2831-41] in 80ml $CH_2Cl_2$ tropft man bei 0-10° im Verlaufe von 30Min. eine Lösung von 15,8g m-Chlorperbenzoesäure in 80ml $CH_2Cl_2$ zu und rührt anschliessend 16Std. bei 20°. Dann verdünnt man mit Ethylacetat, wäscht nacheinander mit 2N wässrigen Lösungen von $K_2CO_3$, $Na_2S_2O_3$ sowie NaCl, trocknet über $Na_2SO_4$ und dampft ein. Dabei erhält man die Titelverbindung, die durch Kristallisation aus $CH_2Cl_2$-Ether-Hexan gereinigt wird.

(b) 2-Chlormethyl-6,7-dimethyl-chinolin: Zu einer Lösung von 6,35g 2,6,7-Trimethylchinolin-1-oxid in 60ml Toluol tropft man unter Rühren bei 50° im Verlaufe von einer Stunde eine Lösung von 9,4ml Benzolsulfochlorid in 10ml Toluol zu und rührt 16Std. bei 50° nach. Dann nimmt man in Ethylacetat auf, wäscht mit 2N wässrigen Lösungen von $NaHCO_3$ sowie NaCl, trocknet über $Na_2SO_4$ und dampft ein. Der Eindampfrückstand wird an 250g Kieselgel chromatographiert. Die produkthaltigen, mit Toluol und Toluol/Ethylacetat 99:1 eluierten Fraktionen werden vereinigt und eingedampft. Dabei erhält man die Titelverbindung, die durch Kristallisation aus $CH_2Cl_2$-Ether-Hexan weiter gereinigt wird.

(c) 3-(6,7-Dimethyl-2-chinolinylmethoxy)anilin: Eine Lösung von 1,51g 3-Aminophenol in 30ml DMF versetzt man bei 0° mit 0,35g NaH (95%) und rührt 30Min. bei 0° nach. Dann gibt man 2,8g 2-Chlormethyl-6,7-dimethyl-chinolin in fester Form dazu und rührt eine weitere Stunde bei 0° sowie nochmals eine Stunde bei 20°. Anschliessend wird mit Ethylacetat verdünnt, mit Wasser gewaschen, über $Na_2SO_4$ getrocknet und eingedampft. Der Eindampfrückstand wird an 250g Kieselgel chromatographiert. Die produkthaltigen, mit Toluol/Ethylacetat 95:5 und 9:1 eluierten Fraktionen werden vereinigt und eingedampft. Dabei erhält man die Titelverbindung, die durch Kristallisation aus $CH_2Cl_2$-Ether weiter gereinigt wird.

Beispiel 23: 4-[3-(7-Methyl-2-chinolinylmethoxy)phenylamino]-2,2-diethyl-4-oxobutansäure

Die Titelverbindung wird analog zu der in Beispiel 18 beschriebenen Verbindung aus 2,2-Diethylbernsteinsäureanhydrid und 3-(7-Methyl-2-chinolinylmethoxy)anilin hergestellt; farblose Kristalle vom Smp. 162-163°.

Die Ausgangsmaterialien werden wie folgt hergestellt:

(a) 2,7-Dimethyl-chinolin-1-oxid: Die Titelverbindung wird analog zu Beispiel 18(a) aus 2,7-Dimethylchinolin [Z. Song et al., J.Heterocyclic Chem. 30 (1993) 17-21] hergestellt; hellbraune Kristalle vom Smp. 65-66°.

(b) 2-Chlormethyl-7-methyl-chinolin: Die Titelverbindung wird analog zu Beispiel 18(b) aus 2,7-Dimethyl-chinolin-1-oxid hergestellt; farblose Kristalle vom Smp. 75-76°.

(c) 3-(7-Methyl-2-chinolinylmethoxy)anilin: Die Titelverbindung wird analog zu Beispiel 18(c) aus 2-Chlormethyl-7-methyl-chinolin und 3-Aminophenol hergestellt; beige Kristalle vom Smp. 97-98°.

Beispiel 24: 4-[3-(7-Methoxy-2-chinolinylmethoxy)phenylamino]-2,2-diethyl-4-oxobutansäure

Die Titelverbindung wird analog zu der in Beispiel 18 beschriebenen Verbindung aus 2,2-Diethylbernsteinsäureanhydrid und 3-(7-Methoxy-2-chinolinylmethoxy)anilin hergestellt; farblose Kristalle vom Smp. 163-164°.

Die Ausgangsmaterialien werden wie folgt hergestellt:

(a) 2-Methyl-7-methoxy-chinolin-1-oxid: Die Titelverbindung wird analog zu Beispiel 18(a) aus 2-Methyl-7-methoxy-chinolin [Z. Song et al., J. Heterocyclic Chem. 30 (1993) 17-21] hergestellt; beige Kristalle vom Smp. 103-104°.

(b) 2-Chlormethyl-7-methoxy-chinolin: Die Titelverbindung wird analog zu Beispiel 18(b) aus 2-Methyl-7-methoxy-chinolin-1-oxid hergestellt; farblose Kristalle vom Smp. 64-65°.

(c) 3-(7-Methoxy-2-chinolinylmethoxy)anilin: Die Titelverbindung wird analog zu Beispiel 18(c) aus 2-Chlormethyl-7-methoxy-chinolin und 3-Aminophenol hergestellt; beige Kristalle vom Smp. 96-97°.

Beispiel 25: 4-[3-(7-Fluor-2-chinolinylmethoxy)phenylamino]-2,2-dimethyl-4-oxobutansäure

Die Titelverbindung wird analog zu der in Beispiel 18 beschriebenen Verbindung aus 2,2-Dimethylbernsteinsäureanhydrid und 3-(7-Fluor-2-chinolinylmethoxy)anilin hergestellt; farblose Kristalle vom Smp. 209-210°.

Beispiel 26: 4-[3-(7-Fluor-2-chinolinylmethoxy)phenylaminol-2-ethyl-2-methyl-4-oxobutansäure

Die Titelverbindung wird analog zu der in Beispiel 18 beschriebenen Verbindung aus 2-Ethyl-2-methylbernsteinsäureanhydrid und 3-(7-Fluor-2-chinolinylmethoxy)anilin hergestellt; farblose Kristalle vom Smp. 179-180°.

Beispiel 27: 1-[3-(7-Fluor-2-chinolinylmethoxy)phenylaminocarbonylmethyl]-cvclopentancarbonsäure

Die Titelverbindung wird analog zu der in Beispiel 18 beschriebenen Verbindung aus (1-Carboxycyclopentyl) essigsäure-anhydrid (erhalten aus der Säure mittels Acetylchlorid analog Beispiel 1) und 3-(7-Fluor-2-chinolinylmethoxy)anilin hergestellt; beige Kristalle vom Smp. 187-188°.

Beispiel 28: 1-[3-(7-Fluor-2-chinolinylmethoxy)phenylaminocarbonylmethyl]cyclohexancarbonsäure

Die Titelverbindung wird analog zu der in Beispiel 18 beschriebenen Verbindung aus (1-Carboxycyclohexyl) essigsäure-anhydrid (erhalten aus der Säure mittels Acetylchlorid analog Beispiel 1) und 3-(7-Fluor-2-chinolinylmethoxy)anilin hergestellt; farblose Kristalle vom Smp. 190-191°.

Beispiel 29: 4-[3-(7-Fluor-2-chinolinylmethoxy)phenylamino]-2,2-dipropyl-4-oxobutansäure

Die Titelverbindung wird analog zu der in Beispiel 18 beschriebenen Verbindung aus 2,2-Dipropylbernsteinsäureanhydrid und 3-(7-Fluor-2-chinolinylmethoxy)anilin hergestellt; beige Kristalle vom Smp. 155-156°.

Beispiel 30: 4-[3-(7-Fluor-2-chinolinylmethoxy)phenylamino]-2,2-dibutyl-4-oxobutansäure

Die Titelverbindung wird analog zu der in Beispiel 18 beschriebenen Verbindung aus 2,2-Dibutylbernsteinsäureanhydrid (hergestellt analog zu Vorschriften von H. Le Moal et al. Bull. Soc. Chim. 1964, 579 und 828) und 3-(7-Fluor-2-chinolinylmethoxy)anilin hergestellt; beige Kristalle vom Smp. 156-157°.

Beispiel 31: 4-[3-(7-Fluor-2-chinolinylmethoxy)phenylamino]-2,2-diethyl-4-oxobutansäure

Die Titelverbindung wird analog zu der in Beispiel 18 beschriebenen Verbindung aus 2,2-Diethylbernsteinsäureanhydrid und 3-(7-Fluor-2-chinolinylmethoxy)anilin hergestellt; farblose Kristalle vom Smp. 166-167°.
Die Ausgangsmaterialien werden wie folgt hergestellt:

(a) 2-Methyl-7-fluor-chinolin-1-oxid: Die Titelverbindung wird analog zu Beispiel 18(a) aus 2-Methyl-7-fluor-chinolin [Z. Song et al., J. Heterocyclic Chem. 30 (1993) 17-21] hergestellt; beige Kristalle vom Smp. 82-83°.

(b) 2-Chlormethyl-7-fluor-chinolin: Die Titelverbindung wird analog zu Beispiel 18(b) aus 2-Methyl-7-fluor-chinolin-1-oxid hergestellt; beige Kristalle vom Smp. 70-71°.

(c) 3-(7-Fluor-2-chinolinylmethoxy)anilin: Die Titelverbindung wird analog zu Beispiel 18(c) aus 2-Chlormethyl-7-fluor-chinolin und 3-Aminophenol hergestellt; beige Kristalle vom Smp. 92-93°.

Beispiel 32: 4-[3-(7-Fluor-2-chinolinylmethoxy)phenylamino]-2,2-diethyl-4-oxobutansäure Natriumsalz (1.4 $H_2O$)

17.65g 4-[3-(7-Fluor-2-chinolinylmethoxy)phenylamino]-2,2-diethyl-4-oxobutansäure werden in 200ml Tetrahydrofuran gelöst und mit 20.8ml einer wässrigen 2n Natriumhydroxidlösung versetzt. Die Lösung wird eingedampft, in Dichlormethan suspendiert und erneut eingedampft. Der Rückstand wird mit 200ml Dichlormethan während 15min am Rückfluss gerührt, abgekühlt und abgesaugt. Die Titelverbindung wird so als farbloses Pulver vom Smp. 118-120° erhalten.

Beispiel 33: 4-[3-(7-Fluor-2-chinolinylmethoxy)phenylamino]-2,2-diethyl-4-oxobutansäure Kaliumsalz (2.6 $H_2O$)

17.65g 4-[3-(7-Fluor-2-chinolinylmethoxy)phenylamino]-2,2-diethyl-4-oxobutansäure werden in 200ml Tetrahydrofuran gelöst und mit 20.8ml einer wässrigen 2n Kaliumhydroxidlösung versetzt. Die Lösung wird eingedampft, in Dichlormethan suspendiert und erneut eingedampft. Der Rückstand wird mit 200ml Dichlormethan während 15min am Rückfluss gerührt, abgekühlt und abgesaugt. Die Titelverbindung wird so als farbloses Pulver vom Smp. 114-116° erhalten.

Beispiele A bis G: Pharmazeutische Präparate

Unter dem Ausdruck "Wirkstoff" ist nachstehend eine Verbindung (I), in freier Form oder in Form eines pharmazeutisch verwendbaren Salzes, zu verstehen, insbesondere eine solche Verbindung, die als Produkt in einem der vorstehenden Beispiele beschrieben ist.

Beispiel A: Eine treibmittelhaltige, feststoffaerosolbildende Inhalationssuspension, enthaltend 0,1 Gewichtsprozent Wirkstoff:

| Zusammensetzung | Gewichtsprozent |
|---|---|
| Wirkstoff, mikronisiert | 0,1 |
| Sorbitantrioleat | 0,5 |
| Treibmittel A (Trichlortrifluorethan) | 4,4 |
| Treibmittel B (Dichlordifluormethan und | 15,0 |
| 1,2-Dichlortetrafluorethan) | 80,0 |

Der Wirkstoff wird unter Feuchtigkeitsausschluss mit Hilfe eines üblichen Homogenisators unter Zusatz des Sorbitantrioleats im Trichlortrifluorethan suspendiert und die Suspension in einen mit einem Dosierventil versehenen Aerosolbehälter abgefüllt. Der Behälter wird verschlossen und unter Druck mit dem Treibmittel B aufgefüllt.

Beispiel B: Eine zur Inhalation geeignete, etwa zweiprozentige, wässrige Lösung des Wirkstoffs in Form seines Natrium- oder Kalium-salzes:

| Zusammensetzung | |
|---|---|
| Wirkstoff (K- oder Na-Salz) | 2000 mg |
| Ethylendiamintetraessigsäure-Dinatriumsalz | 10 mg |
| Benzalkoniumchlorid | 10 mg |
| Wasser, frisch destilliert | ad 100 ml |
| Treibmittel | nach Bedarf |

Der Wirkstoff wird in etwa 60 ml frisch destilliertem Wasser gelöst und der Stabilisator (Ethylendiamintetraessigsäure-Dinatriumsalz) und das Konservierungsmittel (Benzalkoniumchlorid) werden hinzugegeben. Nach vollständiger Auflösung aller Komponenten wird die erhaltene Lösung auf 100 ml aufgefüllt und in Druckfläschchen abgefüllt. Die Fläschchen werden gasdicht verschlossen. Das Treibmittel wird nach Bedarf gasförmig unter Druck oder in flüssiger Form zugegeben.

Beispiel C: Eine Salbe, enthaltend 0,05 Gewichtsprozent Wirkstoff:

| Zusammensetzung | Gewichtsprozent |
|---|---|
| Wirkstoff | 0,05 |
| Vaseline | 45,00 |
| Paraffinöl | 19,60 |
| Cetylalkohol | 5,00 |
| Bienenwachs | 5,00 |
| Sorbitan-sesquioleat | 5,00 |
| p-Hydroxybenzoesäureester | 0,20 |
| Wasser, entmineralisiert | 20,15 |

Die Fettstoffe und Emulgatoren werden zusammengeschmolzen. Das Konservierungsmittel wird in Wasser gelöst und die Lösung in die Fettschmelze bei erhöhter Temperatur einemulgiert. Nach dem Erkalten wird eine Suspension des Wirkstoffs in einem Teil der Fettschmelze in die Emulsion eingearbeitet.

Beispiel D: Tabletten, enthaltend je 50 mg Wirkstoff:

| Zusammensetzung (10000 Tabletten) | |
|---|---|
| Wirkstoff | 500,0 g |
| Lactose | 500,0 g |

(fortgesetzt)

| Zusammensetzung (10000 Tabletten) | |
|---|---|
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Ethanol | q.s. |

Der Wirkstoff wird mit der Lactose und 292 g der Kartoffelstärke vermischt und die Mischung mit einer ethanolischen Lösung der Gelatine befeuchtet und durch ein Sieb granuliert. Nach dem Trocknen mischt man den Rest der Kartoffelstärke, das Magnesiumstearat, den Talk und das Siliciumdioxid zu und presst das Gemisch zu Tabletten von je 145 mg Gewicht und 50 mg Wirkstoffgehalt, die gewünschtenfalls mit Teilkerben zur feineren Anpassung der Dosierung versehen sein können.

Beispiel E: Lacktabletten, enthaltend je 100 mg Wirkstoff:

| Zusammensetzung (1000 Lacktabletten) | |
|---|---|
| Wirkstoff | 100,0 g |
| Lactose | 100,0 g |
| Maisstärke | 70,0 g |
| Talk | 8,5 g |
| Calciumstearat | 1,5 g |
| Hydroxypropylmethylcellulose | 2,36 g |
| Schellack | 0,64 g |
| Wasser | q.s. |
| Dichlormethan | q.s. |

Der Wirkstoff, die Lactose und 40 g der Maisstärke werden gemischt. Das Gemisch wird mit einem Kleister, hergestellt aus 15 g Maisstärke und Wasser (unter Erwärmen), befeuchtet und granuliert. Das Granulat wird getrocknet und der Rest der Maisstärke, der Talk und das Calciumstearat werden mit dem Granulat vermischt. Das Gemisch wird zu Tabletten (Gewicht: je 280 mg) verpresst und diese werden mit einer Lösung der Hydroxypropylmethylcellulose und des Schellacks in Dichlormethan lackiert (Endgewicht der Lacktabletten: je 283 mg).

Beispiel F: Gelatinesteckkapseln, enthaltend je 100 mg Wirkstoff:

| Zusammensetzung (1000 Kapseln) | |
|---|---|
| Wirkstoff | 100,0g |
| Lactose | 250,0 g |
| mikrokristalline Cellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig gemischt. Dann wird zunächst die Lactose durch ein Sieb mit einer Maschenweite von 0,6 mm und anschliessend die mikrokristalline Cellulose durch ein Sieb mit einer Maschenweite von 0,9 mm hinzugesiebt. Dann werden alle vier Komponenten 10 Minuten innig gemischt. Zuletzt wird das Magnesiumstearat durch ein Sieb mit einer Maschenweite von 0,8 mm hinzugesiebt. Nach weiterem Mischen (3 Minuten) werden je 390 mg der erhaltenen Formulierung in Gelatinesteckkapseln der Grösse 0 abgefüllt.

Beispiel G: Eine Injektions- oder Infusionslösung, enthaltend 5 mg Wirkstoff je 2,5 ml-Ampulle:

| Zusammensetzung (1000 Ampullen) | |
|---|---|
| Wirkstoff | 5,0 g |
| Natriumchlorid | 22,5 g |

(fortgesetzt)

| Zusammensetzung (1000 Ampullen) | |
|---|---|
| Phosphatpufferlösung (pH: 7,4) | 300,0 g |
| entmineralisiertes Wasser | ad 2500,0 ml |

Der Wirkstoff und das Natriumchlorid werden in 1000 ml entmineralisiertem Wasser gelöst. Die Lösung wird durch ein Mikrofilter filtriert. Man versetzt das Filtrat mit der Phosphatpufferlösung und füllt das Gemisch mit entmineralisiertem Wasser auf 2500 ml auf. Zur Herstellung von Dosiseinheitsformen werden je 2,5 ml des Gemisches in Glasampullen abgefüllt, die dann je 5 mg Wirkstoff enthalten.

**Patentansprüche**

**1.** Eine Verbindung der Formel I,

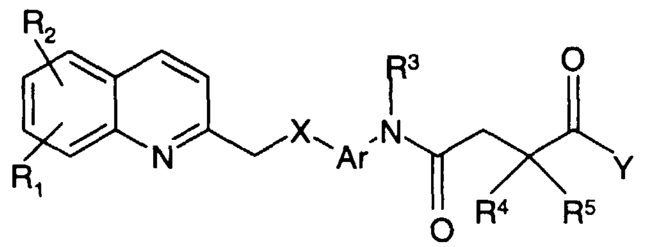

(I)

worin

$R_1$ und $R_2$ unabhängig voneinander Wasserstoff, Niederalkyl, Halogen-Niederalkyl, Halogen, Niederalkoxy, oder Halogen-Niederalkoxy bedeuten, oder

$R_1$ und $R_2$ zusammen $-(CH_2)_m-$, worin m für 3, 4 oder 5 steht, bedeuten,

$R_3$ Wasserstoff, Niederalkyl, oder (Carboxy- oder Niederalkoxycarbonyl-)phenyl-Niederalkyl, welches im Phenylring noch zusätzlich durch Niederalkoxy substituiert sein kann, bedeutet,

$R_4$ und $R_5$ unabhängig voneinander Niederalkyl bedeuten, oder

$R_4$ und $R_5$ susammen $-(CH_2)_n-$, worin n für 3, 4, 5 oder 6 steht, bedeuten,

X für O oder S steht,

Ar für 1,3-Phenylen oder 2,7-Naphthylen steht, und

Y Hydroxy oder Niederalkoxy bedeutet;

worin das Präfix "Nieder" einen Rest bis und mit 7 Kohlenstoffatomen bezeichnet;
oder ein Salz davon.

**2.** Eine Verbindung den Formel I gemäss Anspruch 1, worin das Präfix "Nieder" einen Rest bis und mit 4 Kolenstoffatomen bezeichnet.

**3.** Eine Verbindung der Formel I gemäss Anspruch 1, worin $R_1$ und $R_2$ unabhängig voneinander Wasserstoff, Niederalkyl, Halogen-Niederalkyl, Halogen, Niederalkoxy oder Halogen-Niederalkoxy bedeuten, oder $R_1$ und $R_2$ zusammen $-(CH_2)_3-$ oder $-(CH_2)_4-$ bedeuten, $R_3$ Wasserstoff, Niederalkyl, oder (Carboxy oder $C_1$-$C_7$-alkoxycarbonyl) phenyl Niederalkyl, welches im Phenylring noch zusätzlich durch Niederalkoxy substituiert sein kann, bedeutet, $R_4$ und $R_5$ unabhängig voneinander Niederalkyl bedeuten, oder $R_4$ und $R_5$ zusammen $-(CH_2)_4-$ oder $-(CH_2)_5-$

bedeuten, X für O oder S steht, Ar für 1,3-Phenylen oder 2,7-Naphthylen steht, und Y Hydroxy oder Niederalkoxy bedeutet; oder ein Salz davon.

4. Eine Verbindung der Formel I gemäss Anspruch 1, worin $R_1$ und $R_2$ unabhängig voneinander Wasserstoff, Niederalkyl, Trifluormethyl, Halogen oder Niederalkoxy bedeuten, $R_3$ Wasserstoff, Niederalkyl, oder (Carboxy oder $C_1$-$C_7$-alkoxycarbonyl)phenyl-Niederalkyl bedeutet, $R_4$ und $R_5$ unabhängig voneinander Niederalkyl bedeuten, oder $R_4$ und $R_5$ zusammen $-(CH_2)_4-$ oder $-(CH_2)_5-$ bedeuten, X für O oder S steht, Ar für 1,3-Phenylen oder 2,7-Naphthylen steht, und Y Hydroxy oder Niederalkoxy bedeutet; oder ein Salz davon.

5. Eine Verbindung der Formel I gemäss Anspruch 1, worin $R_1$ und $R_2$ unabhängig voneinander Wasserstoff, Niederalkyl, Trifluormethyl, Halogen oder Niederalkoxy bedeuten, $R_3$ für Wasserstoff, Niederalkyl oder Carboxyphenyl-niederalkyl steht, $R_4$ und $R_5$ unabhängig voneinander Niederalkyl bedeuten, oder $R_4$ und $R_5$ zusammen $-(CH_2)_4-$ oder$-(CH_2)_5-$ bedeuten, X für O oder S steht, Ar für 1,3-Phenylen oder 2,7-Naphthylen steht, und Y Hydroxy oder Niederalkoxy bedeutet, oder ein pharmazeutisch verwendbares Salz davon.

6. Eine Verbindung der Formel I gemäss Anspruch 1, worin $R_1$ Wasserstoff, Niederalkyl, Trifluormethyl, Halogen oder Niederalkoxy bedeutet, $R_2$ und $R_3$ für Wasserstoff stehen, $R_4$ und $R_5$ unabhängig voneinander $C_1$-$C_3$-Alkyl bedeuten, oder $R_4$ und $R_5$ zusammen $-(CH_2)_4-$ oder $-(CH_2)_5-$ bedeuten, X für O oder S steht, Ar für 1,3-Phenylen oder 2,7-Naphthylen steht, und Y Hydroxy bedeutet, oder ein pharmazeutisch verwendbares Salz davon.

7. Eine Verbindung gemäss Anspruch 1 von Formel Ia,

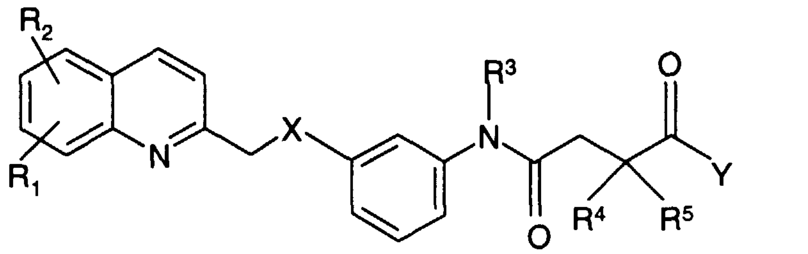

(Ia)

worin $R_1$ und $R_2$ unabhängig voneinander Wasserstoff, Chlor oder Fluor bedeuten, $R_3$ für Wasserstoff steht, $R_4$ und $R_5$ Ethyl bedeuten, X für O steht und Y Hydroxy oder Niederalkoxy bedeutet, oder ein pharmazeutisch verwendbares Salz davon.

8. Eine Verbindung gemäss Anspruch 6 von der Formel Ia, worin $R_1$ Wasserstoff, Chlor oder Fluor bedeutet, $R_2$ für Wasserstoff oder Fluor steht, $R_3$ Wasserstoff bedeutet, $R_4$ und $R_5$ Ethyl bedeuten, X für O steht und Y Hydroxy bedeutet, oder ein pharmazeutisch verwendbares Salz davon.

9. 4-[3-(2-Chinolinylmethoxy)phenylamino]-2,2-diethyl-4-oxobutansäure gemäss Anspruch 1, oder ein pharmazeutisch verwendbares Salz davon.

10. 4-[3-(7-Fluor-2-chinolinylmethoxy)phenylamino]-2,2-diethyl-4-oxobutansäure gemäss Anspruch 1, oder ein pharmazeutisch verwendbares Salz davon.

11. 4-[3-(7-Trifluormethyl-2-chinolinylmethoxy)phenylamino]-2,2-diethyl-4-oxobutansäure gemäss Anspruch 1, oder ein pharmazeutisch verwendbares Salz davon.

**Claims**

1. A compound of formula I,

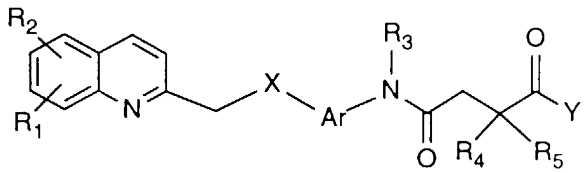

(I)

wherein

$R_1$ and $R_2$ are each independently of the other hydrogen, lower alkyl, halo-lower alkyl, halogen, lower alkoxy or halo-lower alkoxy, or

$R_1$ and $R_2$ together form $-(CH_2)_m-$, wherein m is 3, 4 or 5,

$R_3$ is hydrogen, lower alkyl, or (carboxy- or lower alkoxycarbonyl-)phenyl-lower alkyl, which may be additionally substituted on the phenyl ring by lower alkoxy,

$R_4$ and $R_5$ are each independently of the other lower alkyl, or

$R_4$ and $R_5$ together form $-(CH_2)_n-$, wherein n is 3, 4, 5 or 6,

X is O or S,

Ar is 1,3-phenylene or 2,7-naphthylene, and

Y is hydroxy or lower alkoxy;

the term "lower" denoting a radical having up to and including 7 carbon atoms;
or a salt thereof.

**2.** A compound of formula I according to claim 1, wherein the term "lower" denotes a radical having up to and including 4 carbon atoms.

**3.** A compound of formula I according to claim 1, wherein $R_1$ and $R_2$ are each independently of the other hydrogen, lower alkyl, halo-lower alkyl, halogen, lower alkoxy or halo-lower alkoxy, or $R_1$ and $R_2$ together form $-(CH_2)_3-$ or $-(CH_2)_4-$, $R_3$ is hydrogen, lower alkyl, or (carboxy- or $C_1$-$C_7$alkoxycarbonyl-)phenyl-lower alkyl, which may be additionally substituted on the phenyl ring by lower alkoxy, $R_4$ and $R_5$ are each independently of the other lower alkyl, or $R_4$ and $R_5$ together form $-(CH_2)_4-$ or $-(CH_2)_5-$, X is O or S, Ar is 1,3-phenylene or 2,7-naphthylene, and Y is hydroxy or lower alkoxy; or a salt thereof.

**4.** A compound of formula I according to claim 1, wherein $R_1$ and $R_2$ are each independently of the other hydrogen, lower alkyl, trifluoromethyl, halogen or lower alkoxy, $R_3$ is hydrogen, lower alkyl or (carboxy- or $C_1$-$C_7$alkoxycarbonyl-)pheny]-lower alkyl, $R_4$ and $R_5$ are each independently of the other lower alkyl, or $R_4$ and $R_5$ together form $-(CH_2)_4-$ or $-(CH_2)_5-$, X is O or S, Ar is 1,3-phenylene or 2,7-naphthylene, and Y is hydroxy or lower alkoxy; or a salt thereof.

**5.** A compound of formula I according to claim 1, wherein $R_1$ and $R_2$ are each independently of the other hydrogen, lower alkyl, trifluoromethyl, halogen or lower alkoxy, $R_3$ is hydrogen, lower alkyl or carboxyphenyl-lower alkyl, $R_4$ and $R_5$ are each independently of the other lower alkyl, or $R_4$ and $R_5$ together form $-(CH_2)_4-$ or $-(CH_2)_5-$, X is O or S, Ar is 1,3-phenylene or 2,7-naphthylene, and Y is hydroxy or lower alkoxy, or a pharmaceutically acceptable salt thereof.

**6.** A compound of formula I according to claim 1, wherein $R_1$ is hydrogen, lower alkyl, trifluoromethyl, halogen or lower alkoxy, $R_2$ and $R_3$ are hydrogen, $R_4$ and $R_5$ are each independently of the other $C_1$-$C_3$alkyl, or $R_4$ and $R_5$ together form $-(CH_2)_4-$ or $-(CH_2)_5-$, X is O or S, Ar is 1,3-phenylene or 2,7-naphthylene, and Y is hydroxy, or a pharmaceutically acceptable salt thereof.

**7.** A compound according to claim 1 of formula Ia

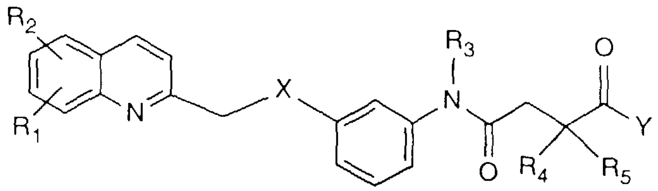

(Ia)

wherein $R_1$ and $R_2$ are each independently of the other hydrogen, chlorine or fluorine, $R_3$ is hydrogen, $R_4$ and $R_5$ are ethyl, X is O, and Y is hydroxy or lower alkoxy, or a pharmaceutically acceptable salt thereof.

**8.** A compound according to claim 6 of formula Ia, wherein $R_1$ is hydrogen, chlorine or fluorine, $R_2$ is hydrogen or fluorine, $R_3$ is hydrogen, $R_4$ and $R_5$ are ethyl, X is O, and Y is hydroxy, or a pharmaceutically acceptable salt thereof.

**9.** 4-[3-(2-Quinolinylmethoxy)phenylamino]-2,2-diethyl-4-oxobutanoic acid according to claim 1, or a pharmaceutically acceptable salt thereof.

**10.** 4-[3-(7-Fluoro-2-quinolinylmethoxy)phenylamino]-2,2-diethyl-4-oxobutanoic acid according to claim 1, or a pharmaceutically acceptable salt thereof.

**11.** 4-[3-(7-Trifluoromethyl-2-quinolinylmethoxy)phenylamino]-2,2-diethyl-4-oxobutanoic acid according to claim 1, or a pharmaceutically acceptable salt thereof.

## Revendications

**1.** Composé de formule I

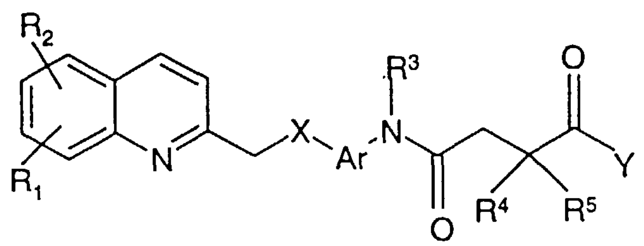

(I)

dans laquelle

| | |
|---|---|
| $R_1$ et $R_2$ | représentent indépendamment l'un de l'autre un hydrogène, un alkyle inférieur, un halogène-alkyle inférieur, un halogène, un alcoxy inférieur ou un halogène-alcoxy inférieur, ou |
| $R_1$et $R_2$ | représentent ensemble $-(CH_2)_m-$, où m vaut 3, 4 ou 5, |
| $R_3$ | représente un hydrogène, un alkyle inférieur, ou un (carboxy- ou alcoxy inférieur-carbonyl-)phényl-alkyle inférieur, qui peut encore être substitué de façon supplémentaire dans le noyau phényle par un alcoxy inférieur, |
| $R_4$ et $R_5$ | représentent indépendamment l'un de l'autre un alkyle inférieur, ou |
| $R_4$ et $R_5$ | représentent ensemble $-(CH_2)_n-$, où n vaut 3, 4, 5 ou 6, |
| X | représente O ou S, |
| Ar | représente un 1,3-phénylène ou un 2,7-naphtylène, et |
| Y | représente un hydroxy ou un alcoxy inférieur ; |

où le préfixe "inférieur" désigne un radical ayant jusqu'à 7 atomes de carbone compris ;
ou un de ses sels.

**2.** Composé de formule I selon la revendication 1, dans laquelle le préfixe "inférieur" désigne un radical ayant jusqu'à 4 atomes de carbone compris.

**3.** Composé de formule I selon la revendication 1, dans laquelle $R_1$ et $R_2$ représentent indépendamment l'un de l'autre un hydrogène, un alkyle inférieur, un halogène-alkyle inférieur, un halogène, un alcoxy inférieur ou un halogène-alcoxy inférieur, ou $R_1$ et $R_2$ représentent ensemble $-(CH_2)_3$-ou $-(CH_2)_4-$, $R_3$ représente un hydrogène, un alkyle inférieur, ou un (carboxy ou alcoxy en $C_1$ à $C_7$-carbonyl)phényl-alkyle inférieur, qui peut encore être substitué de façon supplémentaire dans le noyau phényle par un alcoxy inférieur, $R_4$ et $R_5$ représentent indépendamment l'un de l'autre un alkyle inférieur, ou $R_4$ et $R_5$ représentent ensemble $-(CH_2)_4-$ ou $-(CH_2)_5-$, X représente O ou S, Ar représente un 1,3-phénylène ou un 2,7-naphtylène, et Y représente un hydroxy ou un alcoxy inférieur ; ou un de ses sels.

**4.** Composé de formule I selon la revendication 1, dans laquelle $R_1$ et $R_2$ représentent indépendamment l'un de l'autre un hydrogène, un alkyle inférieur, un trifluorométhyle, un halogène ou un alcoxy inférieur, $R_3$ représente un hydrogène, un alkyle inférieur ou un (carboxy ou alcoxy en $C_1$ à $C_7$-carbonyl)phényl-alkyle inférieur, $R_4$ et $R_5$ représentent indépendamment l'un de l'autre un alkyle inférieur, ou $R_4$ et $R_5$ représentent ensemble $-(CH_2)_4-$ ou $-(CH_2)_5-$, X représente O ou S, Ar représente un 1,3-phénylène ou un 2,7-naphtylène, et Y représente un hydroxy ou un alcoxy inférieur ; ou un de ses sels.

**5.** Composé de formule I selon la revendication 1, dans laquelle $R_1$ et $R_2$ représentent indépendamment l'un de l'autre un hydrogène, un alkyle inférieur, un trifluorométhyle, un halogène ou un alcoxy inférieur, $R_3$ représente un hydrogène, un alkyle inférieur ou un carboxyphényl-alkyle inférieur, $R_4$ et $R_5$ représentent indépendamment l'un de l'autre un alkyle inférieur, ou $R_4$ et $R_5$ représentent ensemble $-(CH_2)_4-$ ou $-(CH_2)_5-$, X représente O ou S, Ar représente un 1,3-phénylène ou un 2,7-naphtylène, et Y représente un hydroxy ou un alcoxy inférieur ; ou un de ses sels pharmaceutiquement utilisables.

**6.** Composé de formule I selon la revendication 1, dans laquelle $R_1$ représente un hydrogène, un alkyle inférieur, un trifluorométhyle, un halogène ou un alcoxy inférieur, $R_2$ et $R_3$ représentent un hydrogène, $R_4$ et $R_5$ représentent indépendamment l'un de l'autre un alkyle en $C_1$ à $C_3$, ou $R_4$ et $R_5$ représentent ensemble $-(CH_2)_4-$ ou $-(CH_2)_5-$, X représente O ou S, Ar représente un 1,3-phénylène ou un 2,7-naphtylène, et Y représente un hydroxy, ou un de ses sels pharmaceutiquement utilisables.

**7.** Composé selon la revendication 1 de formule Ia,

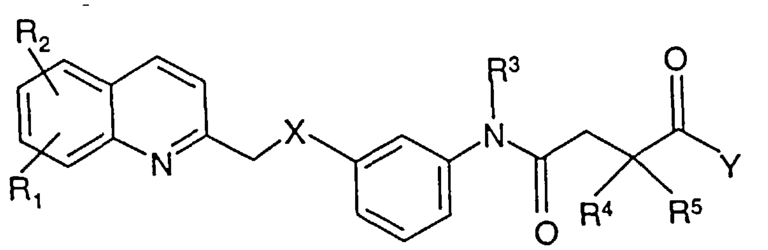

(Ia)

dans laquelle $R_1$ et $R_2$ représentent indépendamment l'un de l'autre un hydrogène, un chlore ou un fluor, $R_3$ représente un hydrogène, $R_4$ et $R_5$ représentent un éthyle, X représente O et Y représente un hydroxy ou un alcoxy inférieur, ou un de ses sels pharmaceutiquement utilisables.

**8.** Composé selon la revendication 6 de formule Ia, dans laquelle $R_1$ représente un hydrogène, un chlore ou un fluor, $R_2$ représente un hydrogène ou un fluor, $R_3$ représente un hydrogène, $R_4$ et $R_5$ représentent un éthyle, X représente O et Y représente un hydroxy, ou un de ses sels pharmaceutiquement utilisables.

**9.** Acide 4-[3-(2-quinoléinylméthoxy)phénylamino]-2,2-diéthyl-4-oxobutanoïque selon la revendication 1, ou un de ses sels pharmaceutiquement utilisables.

**10.** Acide 4-[3-(7-fluoro-2-quinoléinylméthoxy)phénylamino]-2,2-diéthyl-4-oxobutanoïque selon la revendication 1, ou un de ses sels pharmaceutiquement utilisables.

**11.** Acide 4-[3-(7-trifluorométhyl-2-quinoléinylméthoxy)phénylamino]-2,2-diéthyl-4-oxobutanoïque selon la revendication 1, ou un de ses sels pharmaceutiquement utilisables.